# EUROPEAN PATENT APPLICATION

(11) **EP 4 431 617 A1**
(43) Date of publication of application: **18.09.2024**
(21) Application number: 23162720.9
(22) Date of filing: 17.03.2023
(51) Int. Cl.: C12Q 1/6869, C12Q 1/6883

(54) **LONG-READ SEQUENCING OF HUMAN IMMUNOGLOBULIN GENE INTRONS TO DEFINE A BIOMARKER FOR IN VIVO DNA BREAK REPAIR**

(71) Applicant: Max-Delbrück-Centrum für Molekulare Medizin in der Helmholtz-Gemeinschaft, 13125 Berlin (DE); Charité - Universitätsmedizin Berlin, 10117 Berlin (DE)
(72) Inventor: de la Rosa, Kathrin, 10437 Berlin (DE); Vázquez García, Clara, 13357 Berlin (DE); Obermayer-Wasserscheid, Benedikt, 10827 Berlin (DE)
(74) Representative: Moré, Solveig Helga

(57) **Abstract**

The present invention relates to a method for analyzing the DNA break repair machinery of a subject, in particular, for determining at least one deficiency in the DNA break repair machinery, and, on this basis, for predicting the DNA break repair efficiency of the subject. While the analysis is performed in B-cells and comprises analyzing switch-joints of the immunoglobulin heavy chain locus (!GH) of said B-cells, the results can generally be used to predict the risk to develop a cancer, an immunodeficiency such as common variable immunodeficiency (CVID) or a neurodevelopmental disease such as ataxia telangiectasia (AT), for determining the prognosis of a cancer, or for selecting a method of treating a cancer. The invention also relates to a computer program product comprising instructions which, when the program is executed by a computer, cause the computer to carry out specific steps of the analysis, and to a kit comprising said computer program product. The method of the invention is also designated Switch-joint Breakpoint Repertoire Identification (SWIBRID).

## Description

The present invention relates to a method for analyzing the DNA break repair machinery of a subject, in particular, for determining at least one deficiency in the DNA break repair machinery, and, on this basis, for predicting the DNA break repair efficiency of the subject. While the analysis is performed in B-cells and comprises analyzing switch-joints of the immunoglobulin heavy chain locus (IGH) of said B-cells, the results can generally be used to predict the risk to develop a cancer, an immunodeficiency such as common variable immunodeficiency (CVID) or a neurodevelopmental disease such as ataxia telangiectasia (AT), for determining the prognosis of a cancer, or for selecting a method of treating a cancer. The invention also relates to a computer program product comprising instructions which, when the program is executed by a computer, cause the computer to carry out specific steps of the analysis, and to a kit comprising said computer program product. The method of the invention is also designated Switch-joint Breakpoint Repertoire Identification (SWIBRID).

In Germany, 490 000 people per year develop cancer, and the national cancer institute estimates around 1.7 million new cases annually in the US. One crucial issue that can predispose to cancer is genomic instability. However, DNA damage is also utilized for cancer treatment by introducing an excess of lethal mutations through chemotherapy and radiotherapy. On both sides, there is a clinical need to understand the quality of individual DNA break repair to assess: first, the susceptibility for cancer in a person to develop preventative measures, and, second, the impact on successful treatment outcomes by quantifying the DNA damage upon, e.g., radiation dose rates or the use of DNA repair inhibitors.

With the advent of genome engineering techniques for personalized medicine, it has become mandatory to tightly control the repair of induced breaks to reduce dangerous off-target effects. Currently, genome engineering is applied to patients with poor prognoses, but the future holds opportunities for disease prevention. For instance, engineered immune cells ('cellular vaccines') may protect against incurable infectious diseases. Such applications, however, demand a highly effective evaluation of potential side effects by aberrant DNA repair.

CVID is regularly misdiagnosed and confused with, e.g., common allergies or sinusitis (1, 2)). Diagnoses are often made at the age of 20-40, while disease onset typically occurs at age 10. Clinical manifestations have not been clearly defined. Complications of CVID are B-cell lymphoma, chronic enteropathy, inflammatory bowel disease, and cirrhosis. As CVID is diagnosed by exclusion, it can take months to years until available treatments such as life-long intravenous immunoglobulin (IVlg) or subcutaneous immunoglobulin (SCIg) replacement therapy are applied. CVID patients lack B-cell diversity, which critically depends on intact DNA repair machinery. A tool that could identify improper DNA repair early in life could enable long-life monitoring of CVID patients and treatment to avoid complications.

Identifying malfunctions in DNA break repair in humans is thus essential for the early detection of diseases like cancer or immunodeficiencies. The mechanistic pathways of DNA break repair are however partly undefined. DNA break repair is a highly complex process involving multiple pathways. Thus, each individual is expected to have a unique DNA repair profile. Various assays for personalized break repair analysis have been established in the past, but major limitations are the lack of an intracellular environment and randomness of break occurrence across the genome.

In light of this, the inventors have addressed the problem of providing a method for determining DNA break repair efficiency of a subject and a break-repair-footprint that can be used as a biomarker that reflects in vivo DNA repair. Provision of a donor-specific 'break-repair-footprint' is of high clinical value, as it bears predictive power for DNA-repair-associated disease susceptibility, treatment, and prevention.

The invention is defined in the appended claims. In particular, the invention provides a method for determining a DNA break repair deficiency of a subject, comprising steps of
a) providing a sample from the subject comprising B-cells;
b) analyzing switch-joints of the IGH locus of said B-cells, the analysis comprising sequencing the switch-joints using long-read sequencing to generate a library of switch-joint sequencing reads;
c) analyzing the library to determine the DNA break repair footprint and determine a DNA break repair deficiency.

An antibody or immunoglobulin (Ig) is a protein that targets foreign antigens and promotes their elimination by distinct pathways. The antibody comprises 2 immunoglobulin heavy chains and 2 immunoglobulin light chains. The heavy chains are bound to each other by disulfide bonds, as well as each heavy chain to a light chain. Both chains contain a variable and a constant region. Antibodies are generated by B-cells.

In humans, the IGH variable region contains 159 variable (V) domains, 27 diversity (D) domains and 9 junction (J) domains, and the constant region contains exon clusters organized in the order of IGH µ (IGHM), IGH δ (IGHD), IGH γ3 (IGHG3), IGH γ1 (IGHG1), IGH α1 (IGHA1), IGH γ2 (IGHG2), IGH γ4 (IGHG4), IGH ε (IGHE) and IGH α2 (IGHA2) exons, encoded on chromosome 14. The exon clusters encode different immunoglobulin isotypes. Activated B-cells can undergo class switch recombination (CSR), wherein large genomic deletions are introduced in the constant part of the IGH that induce switching from IgM to other immunoglobulin isotypes, namely IgD, IgG, IgA, or IgE. It is an irreversible join between the introns preceding the exons in the IGH, also called deletional recombination. The introns comprise the switch regions. They are characterized by highly repetitive GC-rich sequences. In particular, switch regions are 1-10 kb-long highly repetitive DNA sequences containing RGYW motifs targeted by Activation-induced cytidine deaminase (AID). The exact locations are provided in the table below. In brief, CSR starts with double-stranded DNA (dsDNA) breaks in two switch regions promoted by AID. These breaks come together to be repaired primarily by classical non-homologous end-joining (cNHEJ). During CSR, DNA nicks and dsDNA breaks are repaired by the DNA repair pathways most used by the cells. The most critical players in CSR are germline transcripts (GLTs), AID, and loop extrusion, but a multitude of factors that play a role in dsDNA break repair are also essential here. The dsDNA break resolution gives rise to a switch-joint, also called CSR junction, i.e., the switch-joint is the site in the recombined IGH where the DNA has been rejoined after CSR. Preferably, 100 bp-5 kb, independently of each other, upstream and downstream of the site of recombination are sequenced, more preferably, 200 bp-1 kb upstream and downstream, e.g., about 1000 bp upstream to about 1000 bp downstream, about 500 bp upstream to about 500 bp downstream. Using long-read sequencing, in the context of the invention, means that the majority of reads are at least 500 bp long. Therefore, long passages of the switch-joint regions can be sequenced with the method of the invention, allowing for analysis of CSR breakpoints, sequential switching, intra-switch deletions, and inserts in large stretches of switch-joints.

The inventors considered that, as mechanistic pathways of DNA break repair are partly undefined, the failure of repair processes may be studied by profiling repair outcomes rather than examining the functionality of specific molecular players. However, the infrequent occurrence of breaks at random sites in the genome is challenging for developing disease prediction and prognosis tools. They recognized that the IGH is a sink for DNA break events because activated B-cells undergo CSR that translates to frequently acquiring somatic DNA lesions. Thus, diverse DNA joints created by CSR reflect the relative activity and the quality of DNA repair pathways. The 'DNA break-repair-footprints' gained from diverse activated or, preferably, memory B-cells reflect in vivo DNA repair events and mirror an individual's capability to deal with DNA damage.

The subject typically is a mammal, but the analysis can in principle be carried out for subjects from all species having B-cells undergoing CSR. The subject preferably is a human. It can also be a mouse, a rat, a rabbit, a guinea pig, a goat, a sheep, a camel, or a chicken.

The sample from the subject comprising B-cells comprises activated B-cells or B-cells that have previously been activated, e.g., plasma cells, plasmablasts, or memory B-cells. Preferably, the sample comprises memory B-cells. The sample is typically derived from blood of the subject, but it can also be a sample comprising a germinal center, where B-cells diversify, e.g., a sample comprising a Peyer's patch (PP) or a lymph node. A sample can e.g. comprise peripheral blood lymphocytes (PBL). The DNA of PBL may be isolated directly from blood. The cells may also be purified using a Ficoll gradient. Alternatively or additionally, B-cells or activated B-cells (e.g., plasma cells, plasmablasts and/or memory B-cells) can be isolated based on surface markers, e.g., using fluorescence activated cell sorting (FACS) or magnetic activated cell sorting (MACS), using positive or negative selection. If the concentration of activated B-cells in PBL is low, enrichment of activated B-cells, preferably, memory B-cells is preferred. B-cells can e.g., be enriched using CD19 microbeads.

Based on the sample, in step b), switch-joints of the IGH locus of said B-cells are analyzed, the analysis comprising sequencing the switch-joints using long read sequencing to generate a library of switch-joint sequencing reads.

In a preferred method of the invention, the analysis of step b) comprises, before sequencing, amplification of the switch-joints. Amplification can be performed by a method selected from the group comprising classical PCR and LAM-HTGTS (linear amplification mediated high-throughput genomic translocation sequencing). In the context of the experiments performed herewith, classical PCR was employed, so the details of the method of the invention were optimized in this context. Classical PCR uses two primers, and the sequence between these primers is amplified. Reference herein to PCR without a further specification refers to classical PCR.

A larger-scale approach would be to amplify the switch regions using LAM-HTGTS (4). LAM-HTGTS uses only one primer, which could bind to switch µ region (SM) and amplify downstream in an unbiased manner. This approach is the most unbiased PCR approach to date and can identify large inserts at the DNA level.

An alternative that does not use amplification comprises Cas9 cleavage of DNA, Cas9-assisted targeting of chromosome segments (CATCH). This approach can be addressed using two different ways: i) cutting DNA fragments using two RNAs, one upstream SM and another downstream switch γ region (SGs) and switch α region (SAs); or ii) cutting only one side of the DNA fragment, in this case cutting only upstream SM (e.g., at the same distances as defined herein for primers). PCR biases the amplification of smaller amplicons and can generate point mutations. Despite LAM-HTGTS being the less biased amplification method, it would still commit those mutations. Possibly, PCR errors could create, in some cases, false results. Therefore, a non-PCR-based amplification of the switch-joints using Cas9 fragment enrichment is also possible (5). Cas9 cuts using one or two crRNAs, the extremes of the switch-joints precisely. It also prepares the regions for sequencing, e.g., MinION^{®} sequencing by ligating adapters.

However, the inventors could show that classical PCR is suitable for amplification of switch-joints before sequencing in the method of the invention.

If the amplification is done by a classical PCR, a forward primer annealing to DNA 5' to the switch µ (SM) region and at least one reverse primer annealing to DNA 3' to a switch y1, y2, y3 and/ or y4 (SG1, SG2, SG3, SG4) region and/or 3' the switch α1 (SA1) and/or switch α2 (SA2) region are used. The distance of the primers from the SM, SG or SA region is selected such that it allows for amplification of the DNA between the primers if a recombination at the respective regions has occurred. If no recombination has occurred, the distance is more than 80 kb (SM-SG3), which is far beyond any PCR amplification limit. The PCR thus automatically selects switch-joints for amplification. The length of amplicons can be, e.g., up to 5 kb. Typically, the primer sites are selected to be at most 2 kb, preferably, at most 1 kb from the respective switch region. In this context, 5' or 3' denotes the direction of transcription of an Ig from the locus.

**Table X1: Switch regions:**

| **Name** | **Specie** | **GenBank or reference** | **Length (bp)** |
|---|---|---|---|
| **Switch mu** | Human | X54713.1 | 4525 |
| **Switch gamma 3** | Human | U39935.1 | 3395 |
| **Switch gamma 1** | Human | U39737.1 | 4171 |
| **Switch alpha 1** | Human | L19121.1 | 3326 |
| **Switch gamma 2** | Human | U39934.1 | 3812 |
| **Switch gamma 4** | Human | X56796.1 | 4757 |
| **Switch alpha 2** | Human | AF030305.1 | 2758 |
| **Switch mu** | Mouse | AF446347.1 | 870 |
| **Switch gamma 3** | Mouse | D78343.1 | 1801 |
| **Switch gamma 1** | Mouse | M12389.2 | 5188 |
| **Switch gamma 2b** | Mouse | (*6*) | 4176 |
| **Switch gamma 2c** | Mouse | (*6*) | 2104 |
| **Switch alpha** | Mouse | D11468.1 | 3578 |

SM, SG or SA regions of different species have been defined in the art (e.g., human switch µ region (Genbank: X54713.1) or mouse switch γ2 region (6), as shown in Table X1), and these definitions can be applied. However, the method of the invention preferably uses the coordinates of Table X2 as switch regions, because the inventors observed that the switch regions annotated by the literature were too narrow to cover all the breakpoints observed. Therefore, the switch regions were redefined. Switch region coordinates are used during filtering. Those reads that do not align to any of these regions are discarded. Some regions cover exon sequences.

| Switch region | Specie | Chromosome | Coordinates | Assembly | Exon? |
|---|---|---|---|---|---|
| SM | Human | 14 | 106322600-106328000 | GRCh37 | NO |
| SG3 | Human | 14 | 106238000-106241700 | GRCh37 | NO |
| SG1 | Human | 14 | 106209000-106213800 | GRCh37 | YES |
| SA1 | Human | 14 | 106174500-106179600 | GRCh37 | YES |
| SG2 | Human | 14 | 106111400-106115000 | GRCh37 | NO |
| SG4 | Human | 14 | 106093000-106095700 | GRCh37 | NO |
| SE | Human | 14 | 106068712-106069622 | GRCh37 | NO |
| SA2 | Human | 14 | 106055000-106058700 | GRCh37 | NO |
| SM | Mouse | 12 | 113423700-113426700 | GRCm38 | YES |
| SG3 | Mouse | 12 | 113361000-113366200 | GRCm38 | YES |
| SG1 | Mouse | 12 | 113330500-113341900 | GRCm38 | YES |
| SG2b | Mouse | 12 | 113307500-113314800 | GRCm38 | YES |
| SG2c | Mouse | 12 | 113288400-113294800 | GRCm38 | YES |
| SE | Mouse | 12 | 113273200-113276500 | GRCm38 | YES |
| SA | Mouse | 12 | 113260500-113265700 | GRCm38 | NO |
| Table X2: Description of preferred switch regions used by SWIBRID. The definition of the regions was performed upon observation of breakpoint occurrence in such areas. | | | | | |

Preferred primers and zones against which primers can be generated are provided in the table below for human (GRCh37/38) and murine (GRCm38/39) subjects. In column "type" there are three preferred zones to create the primer: primer (preferred primers), zone 500 nt and zone 1000 nt. For example, the SM forward primer can be located in the region of: chr14:106326994 - 106327493 (500 nt region) or chr14:106326818 - 106327817 (1000 nt region) (GRCh37).

For SG primers may e.g., bind in the following regions:
GRCh37 - SG1
500 nt region: chr14:106210019 - 106210518
1000 nt region: chr14:106209667 - 106210666
GRCh37 - SG2
500 nt region: chr14:106111416 - 106111915
1000 nt region: chr14:106111127 - 106112126
GRCh37 - SG3
500 nt region: chr14:106238208 - 106238707
1000 nt region: chr14:106237974 - 106238973
GRCh37 - SG4
500 nt region: chr14:106092989 - 106093488
1000 nt region: chr14:106092708 - 106093707

The SA primer may, e.g., bind in the following regions:
GRCh37 - SA1
500 nt region: chr14:106175016 - 106175515
1000 nt region: chr14:106175002 - 106176001
GRCh37 - SA2
500 nt region: chr14:106054846 - 106055345
1000 nt region: chr14:106054732 - 106055731

The skilled person can select primers so that, e.g., amplicons having a length up to 10 kb, up to 5 kb, up to 4 kb, up to 2 kb or up to 1 kb are generated. A preferred mean length of amplicons is about 2.5 kb.

| **Name** | **Coordinates** | **Sequence** | **Chromos ome** | **Type** | **Orientation** | **Assembly** |
|---|---|---|---|---|---|---|
| SM | 105860975 - 105861001 | CACCCTTGAAAGTAG CCCATGCCTTCC (SEQ ID NO: 1) | 14 | Primer | forward | GRCh38 |
| | 106327185 - 106327211 | | | | | GRCh37 |
| SM | 105856877 - 105856902 | GGAACGCAGTGTAGA CTCAGCTGAGG (SEQ ID NO: 5) | 14 | Primer | reverse | GRCh38 |
| | 106322982 - 106323007 | | | | | GRCh37 |
| SA | 105588857 - 105588879 | CTCAGTCCAACACCC ACCACTCC (SEQ ID NO: 2) | 14 | Primer | reverse | GRCh38 |
| | 105709106 - 105709128 | | | | | |
| | 106055194 - 106055216 | | | | | GRCh37 |
| | 106175443 - 106175465 | | | | | |
| SG | 105626824 - 105626852 | | 14 | Primer | reverse | GRCh38 |
| | 105645549 - 105645577 | | | | | |
| | 105743825 - 105743853 | | | | | |
| | 105772166 - 105772194 | | | | | |
| | 106093161 - 106093189 | | | | | GRCh37 |
| | 106111886 - 106111914 | | | | | |
| | 106210162 - 106210190 | | | | | |
| | 106238503-106238531 | | | | | |
| SE | 105601669 - 105601692 | | 14 | Primer | reverse | GRCh38 |
| | 106068006 - 106068029 | | | | | GRCh37 |
| SM | 113426178 - 113426200 | GGAGGGACCCAGGCT AAGAAGGC (SEQ ID NO: 6) | 12 | Primer | forward | GRCm38 |
| | 113389798-113389820 | | | | | GRCm39 |
| SA | 113260804 - 113260830 | GCAAGCAGTGGACCC AAAGACGAGAGG (SEQ ID NO: 7) | 12 | Primer | reverse | GRCm38 |
| | 113224424 - 113224450 | | | | | GRCm39 |
| SG1 | 113330330 - 113330354 | | 12 | Primer | reverse | GRCm38 |
| | 113293950 - 113293974 | | | | | GRCm39 |
| SG3 | 113361218 - 113361244 | | 12 | Primer | reverse | GRCm38 |
| | 113324838 - 113324864 | | | | | GRCm39 |
| SG2 | 113307913 - 113307936 | | 12 | Primer | reverse | GRCm38 |
| | 113288916 - 113288936 | | | | | |
| | 113271533 - 113271556 | | | | | GRCm39 |
| | 113252536 - 113252556 | | | | | |
| SE | 113273349 - 113273369 | | 12 | Primer | reverse | GRCm38 |
| | 113236969 - 113236989 | | | | | GRCm39 |
| SM | 105860784 - 105861283 | | 14 | Zone 500 nt | forward | GRCh38 |
| | 106326994 - 106327493 | | | | | GRCh37 |
| SM | 105856487 - 105856986 | | 14 | Zone 500 nt | reverse | GRCh38 |
| | 106322592 - 106323091 | | | | | GRCh37 |
| SA1 | 105708679 - 105709178 | | 14 | Zone 500 nt | reverse | GRCh38 |
| | 106175016 - 106175515 | | | | | GRCh37 |
| SA2 | 105588509 - 105589008 | | 14 | Zone 500 nt | reverse | GRCh38 |
| | 106054846 - 106055345 | | | | | GRCh37 |
| SG1 | 105743682 - 105744181 | | 14 | Zone 500 nt | reverse | GRCh38 |
| | 106210019 - 106210518 | | | | | GRCh37 |
| SG3 | 105771871 - 105772370 | | 14 | Zone 500 nt | reverse | GRCh38 |
| | 106238208 - 106238707 | | | | | GRCh37 |
| SG2 | 105645079 - 105645578 | | 14 | Zone 500 nt | reverse | GRCh38 |
| | 106111416 - 106111915 | | | | | GRCh37 |
| SG4 | 105626652 - 105627151 | | 14 | Zone 500 nt | reverse | GRCh38 |
| | 106092989 - 106093488 | | | | | GRCh37 |
| SE | 105601903 - 105602402 | | 14 | Zone 500 nt | reverse | GRCh38 |
| | 106068240 - 106068739 | | | | | GRCh37 |
| SM | 113426097 - 113426596 | | 12 | Zone 500 nt | forward | GRCm38 |
| | 113389717 - 113390216 | | | | | GRCm39 |
| SA | 113260422 - 113260921 | | 12 | Zone 500 nt | reverse | GRCm38 |
| | 113224042 - 113224541 | | | | | GRCm39 |
| SG1 | 113330328 - 113330827 | | 12 | Zone 500 nt | reverse | GRCm38 |
| | 113293948 - 113294447 | | | | | GRCm39 |
| SG3 | 113361119 - 113361618 | | 12 | Zone 500 nt | reverse | GRCm38 |
| | 113324739 - 113325238 | | | | | GRCm39 |
| SG2b | 113307917 - 113308416 | 12 | Zone 500 nt | reverse | GRCm38 | |
| | 113271537 - 113272036 | | | | GRCm39 | |
| SG2c | 113288916 - 113289415 | 12 | Zone 500 nt | reverse | GRCm38 | |
| | 113252536 - 113253035 | | | | GRCm39 | |
| SE | 113273250 - 113273749 | 12 | Zone 500 nt | reverse | GRCm38 | |
| | 113236870 - 113237369 | | | | GRCm39 | |
| SM | 105860608 - 105861607 | 14 | Zone 1000 nt | forward | GRCh38 | |
| | 106326818 - 106327817 | | | | GRCh37 | |
| SM | 105856218 - 105857217 | 14 | Zone 1000 nt | reverse | GRCh38 | |
| | 106322323 - 106323322 | | | | GRCh37 | |
| SA1 | 105708665 - 105709664 | 14 | Zone 1000 nt | reverse | GRCh38 | |
| | 106175002 - 106176001 | | | | GRCh37 | |
| SA2 | 105588395 - 105589394 | 14 | Zone 1000 nt | reverse | GRCh38 | |
| | 106054732 - 106055731 | | | | GRCh37 | |
| SG1 | 105743330 - 105744329 | 14 | Zone 1000 nt | reverse | GRCh38 | |
| | 106209667 - 106210666 | | | | GRCh37 | |
| SG3 | 105771637 - 105772636 | 14 | Zone 1000 nt | reverse | GRCh38 | |
| | 106237974 - 106238973 | | | | GRCh37 | |
| SG2 | 105644790 - 105645789 | 14 | Zone 1000 nt | reverse | GRCh38 | |
| | 106111127 - 106112126 | | | | GRCh37 | |
| SG4 | 105626371 - 105627370 | 14 | Zone 1000 nt | reverse | GRCh38 | |
| | 106092708 - 106093707 | | | | GRCh37 | |
| SE | 105601728 - 105602727 | 14 | Zone 1000 nt | reverse | GRCh38 | |
| | 106068065 - 106069064 | | | | GRCh37 | |
| SM | 113425949 - 113426948 | 12 | Zone 1000 nt | forward | GRCm38 | |
| | 113389569 - 113390568 | | | | GRCm39 | |
| SA | 113260238 - 113261237 | 12 | Zone 1000 nt | reverse | GRCm38 | |
| | 113223858 - 113224857 | | | | GRCm39 | |
| SG1 | 113330329 - 113331328 | 12 | Zone 1000 nt | reverse | GRCm38 | |
| | 113293949 - 113294948 | | | | GRCm39 | |
| SG3 | 113361217 - 113362216 | 12 | Zone 1000 nt | reverse | GRCm38 | |
| | 113324837 - 113325836 | | | | GRCm39 | |
| SG2b | 113307918 - 113308917 | 12 | Zone 1000 nt | reverse | GRCm38 | |
| | 113271538- 113272537 | | | | GRCm39 | |
| SG2c | 113288916 - 113289915 | 12 | Zone 1000 nt | reverse | GRCm38 | |
| | 113252536 - 113253535 | | | | GRCm39 | |
| SE | 113273250 - 113274249 | 12 | Zone 1000 nt | reverse | GRCm38 | |
| | 113236870 - 113237869 | | | | GRCm39 | |
| Table X3. Description of areas to design primers for analysis of switch region joints in human (GRCh38 assembly) and mouse (GRCm38 assembly) classified in the column "type" by preference. | | | | | | |

In a human subject, reverse primers will always bind 3' to all SG or SA regions, as the respective sequences are the same. In a preferred embodiment, the subject is a human and three primers are used:
a) a forward primer annealing to DNA 5' to the SM region, wherein, optionally, the primer comprises SEQ ID NO: 1 (CACCCTTGAAAGTAGCCCATGCCTTCC );
b) a reverse primer annealing to DNA 3' to the SG1, SG2, SG3 and SG4 regions, wherein, optionally, the primer comprises SEQ ID NO: 3 (CTGCCTCCCAGTGTCCTGCATTACTTCTG); and,
c) a reverse primer annealing to DNA 3' to the SA1 and SA2 region, wherein, optionally, the primer comprises SEQ ID NO: 2 (CTCAGTCCAACACCCACCACTCC).

If it is desired to control for presence of naive B-cells that have not undergone CSR, a SM reverse primer can also be included that binds 3' to the SM region, e.g., the primer of SEQ ID NO: 5. This can control if the PCR worked at all, but will not show class switch. This primer is typically not used.

Optionally, a SE reverse primer, i.e., a reverse primer annealing to DNA 3' to the SE region can be included in addition to the reverse primer annealing to DNA 3' to the SG1, SG2, SG3 and SG4 regions, and, the reverse primer annealing to DNA 3' to the SA1 and SA2 region, e.g., the primer of SEQ ID NO: 4. In the examples below, it is shown that the analysis of SE regions is typically not required to obtain good results.

To allow for multiplexing of sequencing reactions, one primer, or, in the human system, preferably two primers may optionally further comprise one barcode region. Of course, the primers comprising a barcode sequence must still be suitable for amplifying the switch-joints of interest. Barcoding is not required in case multiplexing is not of interest. Methods for barcoding are known in the art and described herein.

The inventors could show that, in the murine system, it was advantageous to use only one barcoded primer, e.g., a barcoded mouse SM (mSM) forward primer for PCR. If the B-cells are murine, it is preferred that the analysis of the IGH locus is limited to SM-SA, optionally also SM-SG3 and SM-SG2b.c switch-joints. Optionally, barcoded mSM forward and unbarcoded SA/SG2b.c/SG3 reverse primer can be employed for amplification of switch-joints from mouse B-cells.

In one embodiment, the amplification of switch regions using gDNA may be further improved by placing the SG reverse and SA reverse primers in the exons rather than switch regions (7). Unlike switch regions, the exons are not targeted by AID during CSR. Thus, exons will always be present in the IGH locus and using them to place primers would ensure that even when the break occurs very close to the exon, a sustainable amount of amplicons can still be amplified. In this approach, the PCR would need to amplify a longer stretch of DNA.

In the method of the invention, after amplification or cutting with Cas9, samples are prepared for sequencing, e.g., as known in the art. For example, amplicons may be purified, e.g., using beads. The DNA can be quality controlled, and a library for sequencing prepared, e.g., as laid out in the manufacturer's instructions. However, it is preferred not to use DNA control in preparation of the library.

Long amplicons can be sequenced using long-read sequencing (L-NGS) devices, e.g., MinION^{®} or PacBio^{®}, or, alternatively by next-generation sequencing such as Illumina^{®} or Ion Torrent sequencing upon fragmentation of amplicons. The latter generates highly accurate reads with a maximum read length of 2x300 bp. However, short reads demand reconstitution and assembly of amplicons using computational tools. Considering that switch regions are characterized by long, repetitive GC-rich sequences, accurate reconstitution of fragmented switch-joint amplicons is excluded. Thus, the inventors decided to sequence amplicons using an L-NGS device. Sequencing is thus preferably performed by a third-generation sequencing method selected from the group comprising
a) nanopore sequencing such as MinION^{®} sequencing and
b) single-molecule real-time (SMRT) sequencing such as PacBio^{®} sequencing,
preferably, nanopore sequencing.

Using nanopore sequencing, a single molecule of DNA or RNA can be sequenced without the need for PCR amplification or chemical labeling of the sample. Nanopore sequencing has the potential to offer relatively low-cost genotyping, high mobility for testing, and rapid processing of samples with the ability to display results in real-time. Biological nanopore sequencing relies on the use of transmembrane proteins, called protein nanopores, in particular, formed by protein toxins, that are embedded in lipid membranes so as to create size dependent porous surfaces - with nanometer scale "holes" distributed across the membranes. Alternatively, solid state nanopores uses metal or metal alloy substrates with nanometer sized pores that allow DNA or RNA to pass through. The nucleic acid is translocated through the pore via a combination of electro-phoretic, electro-osmotic and sometimes thermo-phoretic forces. The magnitude of the electric current density across a nanopore surface depends on the nanopore's dimensions and the composition of DNA or RNA that is occupying the nanopore. Sequencing was made possible because, passing through the channel of the nanopore, the samples cause characteristic changes in the density of the electric current flowing through the nanopore.

SMRT sequencing is a parallelized single molecule DNA sequencing method. Single-molecule real-time sequencing utilizes a zero-mode waveguide (ZMW). A single DNA polymerase enzyme is affixed at the bottom of a ZMW with a single molecule of DNA as a template. The ZMW is a structure that creates an illuminated observation volume that is small enough to observe only a single nucleotide of DNA being incorporated by DNA polymerase. Each of the four DNA bases is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call is made according to the corresponding fluorescence of the dye.

SMRT sequencing such as PacBio^{®} gives rise to more accurate reads than MinION^{®} (error rate <1% versus 5%, respectively), but it is also known to give rise to a lesser read yield. MinION^{®} is a more flexible technology than PacBio^{®}, since MinION^{®} can sequence without the need of PCR amplification. The inventors decided to use MinION^{®} despite its 5% error rate, which was tackled bioinformatically (see below).

Sequencing provides a library of switch-joint sequencing reads. Step c) of the method of the invention comprises analyzing the library to determine the DNA break repair footprint and determine a DNA break repair deficiency.

The analysis of the method of the invention preferably comprises i) filtering reads that do not match quality criteria, ii), optionally, identifying inserts, iii) measuring the differences between reads and iv) determining the number of individual B-cell clones or, preferably, the number of effective individual B-cell clones in a sample.

Preferably, in analysis of step c) of the invention, most preferably, before further analysis is carried out, sequencing reads are filtered and reads not meeting quality criteria are discarded. Preferably, at least 3, preferably at least 4, at least 5 or all of the following criteria apply:
i. Reads are longer than 500 nt;
ii. The sequence of the read must contain at least a 20 nucleotide sequence of one switch region;
iii. The sequence of the read must align in more than 90% of its length to the genome (of course, of the same species, i.e., if the subject is human, a human genome is aligned and/or,
iv. Reads must follow B-cell class switch rules;
   and/or, in case the analysis of step b) comprises, before sequencing, amplification of the switch-joints by PCR,
v. Reads contain a SM forward primer sequence and a reverse primer sequence within 100 nt from the beginning and the end of the read, respectively; wherein the primers preferably have SEQ ID NO: 1, 2 and 3; and/or
vi. Reads should not contain single primers outside 100 nt from the beginning or the end of the read.

For instance, the sequence of the read should contain at least part of one switch region. The minimum size of the switch region that a read needs to contain is determined by the mapping algorithm. For example, by default, LAST only reports significant alignments that will rarely occur by chance. For example, the minimum alignment length may be about 20 nucleotides, preferably, at least 25 nucleotides, optionally, at least 50 nucleotides.

Typically, acceptable reads either map with more than 90% to at least one (typically, two) switch region(s), or map to a switch region, a different region of the genome, and a switch region again. If more than 50 nt of the sequence flanked by switch region sequence does not align to switch regions, the read will be considered to comprise an insert as long as its sequence aligns in at least 95% of its length to the genome.

In particular, at least one, preferably, both following B-cell class-switch biology rules should be followed:
- A switch-joint can include more than two different switch regions. B-cells can class-switch more than one time, due to a process called sequential switching; and/or
- Class-switching can only occur following the (-) sense direction of the IGH locus due to the intragenetical deletional recombination. In other words, if the SM-SG1 joint occurs, SG3 should not appear after SG1, or it would be considered an insert.

Further, if two primers are found in the middle of the read, the read can be split between said primer sequences and considered as two independent reads. This improves the read yield.

So far, in the art, in analysis of CSR in B-cells, the number of individual B-cell clones in the sample was not determined, but the number of reads was considered the number of B-cell clones (8). In contrast, the inventors have found it advantageous if in step c), the number of individual B-cell clones in the sample is determined by clustering of sequencing reads, and the number of clusters is considered to represent the number of individual B-cell clones in the sample.

The distance measure computes the differences between reads. In other words, it compares how similar reads are to each other, using read alignments to a reference genome (of course, of the same species).

In the analysis of the method of the invention, preferably, alignments of reads to a reference genome are transformed into coverage patterns. These coverage patterns may be clustered, preferably, using hierarchical agglomerative clustering. This can be visualized in a dendrogram.

Advantageously, similarities between coverage patterns for different reads are analyzed to provide clusters, optionally based on Jaccard or Cosine distance, preferably, Cosine distance.

A combination of two measurements is also possible i) the interval distance that considers the intersection and union of alignment blocks (using Jaccard distance) and ii) the switch overlap distance that compares coverages of each of the switch regions in reads. Both may be calculated and weighted equally to calculate the differences between the reads in a sample. As a result, a dendrogram of read differences can be generated.

Optionally, for the clustering analysis, gaps in the alignment smaller than 75 bp are ignored.

The number of clusters may be determined by controlling the maximum "cophenetic" (intra-cluster) distance, e.g., from an inflection point in a curve showing either the cluster number or the entropy of the cluster size distribution at different distance cut-offs.

Preferably, the smallest number of clusters that contain only 90-99%, e.g., 92-98%, 93-97%, 94-96% preferably, about 95% of reads are further analyzed. Alternatively, only the largest clusters with the smallest inter-cluster distances containing about 95% of reads are further analyzed. The number of these clusters is considered to represent the number of effective individual B-cell clones in the sample.

The inventors could show that by identifying the number of effective individual B-cell clones (eff_nclusters), the additional diversification caused by MinION^{®} mutations was avoided. Also, they successfully determined that the number of reads comprising a sample does not play a role in eff_nclusters identification. In addition, the add-on mutations by MinION^{®} did not affect the analysis carried out this way.

In a preferred embodiment, the method of the invention
- discards reads that do not meet quality criteria,
- Identifies breakpoints of switch-joints, inserts and/or Ig isotypes,
- clusters reads and determines the number of effective individual B-cell clones.

The examples below show different methods of analyzing the library of reads, e.g., using graphical means such as a two-dimensional breakpoint histogram (2D-bps plot or histogram) showing the frequency of switch-joints between donor and acceptor with donor switch region coordinates on one axis and acceptor switch region coordinates on the other axis, wherein, optionally, machine learning is carried out considering every position in the 2D-bps histogram as a feature, and/or principal components analysis, which can be employed in the method of the invention.

However, in a preferred embodiment, the analysis of step c) of the method of the invention comprises using machine learning to classify a pattern of features. Preferably, these features are derived from clustered read coverage patterns.

Exemplary features that may be used are selected from the group comprising

| |
|---|
| Donor_score_TCCA: average frequency of TCCA sequence motifs around donor switch-joints |
| eff_nclusters: smallest number of clusters comprising at least 95% of all filtered reads |
| donor_score_TG: average frequency of TG sequence motifs around donor switch-joints |
| donor_score_GC: average frequency of GC sequence motifs around donor switch-joints |
| donor_score_AT average frequency of AT sequence motifs around donor switch-joints |
| mean_lenqth: mean length of clustered coverage patterns |
| donor_score_CAGCT: average frequency of CAGCT sequence motifs around donor switch-joints |
| frac_SA: fraction of SA switch-joints |
| donor_score_CA: average frequency of CA sequence motifs around donor switch-joints |
| mean_GC: mean GC content of clustered coveraqe patterns |
| num_mutated pos: number of positions with mutations relative to the reference genome |
| SM_downstream_bias: average entropy of receiver isotype distribution in SM bins relative to overall distribution |
| receiver_score_CA: average frequency of CA sequence motifs around receiver switch-joints |
| cluster_inverse_simpson: inverse Simpson index of cluster size distribution |
| donor_score_CAGCC: average frequency of CAGCC sequence motifs around donor switch-joints |
| std_lenqth: standard deviation of the length of clustered coverage patterns |
| spread_SA: standard deviation of the distribution of SA switch-joint position |
| receiver_score_TG: average frequency of TG sequence motifs around receiver switch-joints |
| loq10_inversions: log10 of the number of sequence inversions |
| log10_nreads: log10 of the number of clustered reads |

Optionally, at least 6, at least 8, at least 9, preferably, at least 10 features are selected from said group of features. Of course, other features can also be used.

In one embodiment of the method of the invention, in step c), the analysis comprises provision of a 2D-bps plot showing the donor switch region on one axis and the acceptor switch region on the other axis, wherein, optionally, machine learning is carried out considering every position in the 2D-bps plot as a feature.

Machine learning can comprise, e.g., i) logistic regression (LR), ii) support vector classifier (SVC) or iii) random forest (RF).

The analysis of step c) can be performed by characterizing the breakpoint profiles of switch-joints and characterizes them using up to 100 different features. To use SWIBRID more effectively, the inventors decided to pinpoint the most important features to identify genotypes and avoid comparing about 90 features between the samples.

The 20 most helpful features are listed in the table above, in the order of their contribution to accurately identify the genotype.

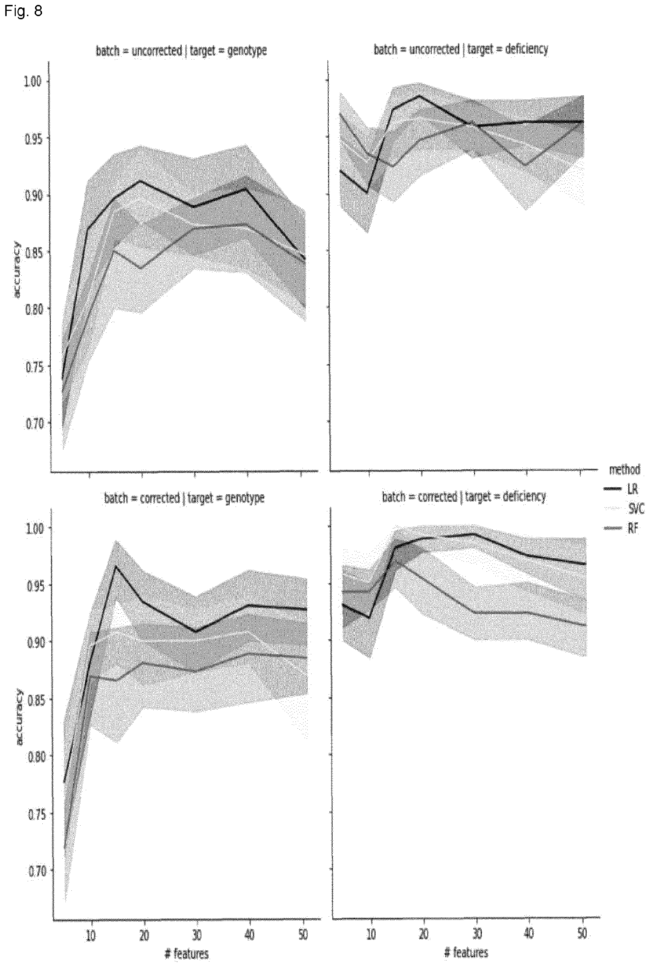

Three different machine learning approaches were used to determine the number of features necessary to differentiate the genotypes using machine learning i) logistical regression (LR), ii) support vector classifier (SVC) and iii) random forest (RF). As shown in Fig. 8, the optimal number of features to identify the genotypes is between 15 and 20 when using them in the order of the ranking. Also, similar results were obtained when the identification was focused on determining the sample as wildtype (WT) or disease. The highest accuracy was observed when using LR.

In conclusion, the analysis shows that, it may be advantageous to perform the analysis using less than 20 features, e.g., between 15 and 20 features. LR may be ideal for the analysis.

The features found in the library can be considered to form a DNA-break repair footprint of the subject. Said DNA-break repair footprint can be used to gain further information, e.g., for a diagnosis, for determining a prognosis or selection of a therapy.

For further analysis of, the method of the present invention may comprise comparing the DNA-break repair footprint of the subject with the DNA-break repair footprint of at least one control sample having a deficiency in the DNA-break repair machinery, wherein the deficiency is selected from the group comprising AID deficiency, AT mutated (ATM) deficiency, Breast cancer gene 1 (BRCA1) deficiency, Breast cancer gene 2 (BRCA2) deficiency, Ligase 4 deficiency, RAD51 Recombinase paralog C (RAD51C) deficiency, RAD51 Recombinase paralog D (RAD51D) deficiency, Partner and localizer of BRCA2 (PALB2) deficiency, BRCA1 Associated RING Domain 1 (BARD1) deficiency, excision repair cross complementation group 1 (ERCC1) deficiency, Artemis deficiency, recombination-activating gene (RAG) deficiency, DNA-dependent protein kinase, catalytic subunit (DNA-PKcs) deficiency, Phosphatase and tensin homolog (PTEN) deficiency, X-ray repair cross-complementing protein 4 (XRCC4) deficiency, X-ray repair cross-complementing protein 2 (XRCC2) deficiency, X-ray repair cross-complementing protein 1 (XRCC1) deficiency, DNA polymerase δ deficiency, Ligase 1 deficiency, Ligase 3 deficiency, XRCC4-like factor (XLF) deficiency, ERCC excision repair 6 like 2 (ERCC6L2) deficiency, Proliferating cell nuclear antigen (PCNA) deficiency, Lynch syndrome, p53 binding protein 1 (53BP1) deficiency and NIPBL deficiency.

Preferably, the deficiency is in the dsDNA break repair machinery. It is plausible that deficiencies in the other components of the DNA repair machinery can also be determined, because not all the molecular players of the DNA repair mechanisms have been yet identified. It is plausible that malfunction in the mismatch repair (MMR) DNA repair pathway, e.g., Lynch syndrome; single strand break repair (SSB), e.g., Topoisomerase I (TOP1), base excision repair (BER) also called indirect SSB, e.g., uracil-DNA glycosylase (UNG); nucleotide excision repair (NER), e.g., damage specific DNA binding protein 1 (DBB1); classical non-homologous end joining (cNHEJ), e.g., Ligase 4; alternative end-joining (aEJ) also called microhomology end-joining (MMEJ), e.g., Ligase III; homologous recombination (HR), e.g., BRCA1; single strand annealing (SSA), e.g., RAD52; are identified by the method of the invention, SWIBRID. In the context of the present application "deficiency" is used synonymously with "malfunction". A deficiency in the DNA break repair machinery can be caused by a lack of protein or a loss of function, but it may also be caused by a gain of function mutation that has disadvantageous effects on DNA break repair in the subject.

The control sample, or, preferably, control samples, can be derived from a subject or a cohort of subjects having such a deficiency. Alternatively, or additionally, the control sample can be generated, e.g., using cells in which the deficiency has been artificially generated, e.g., in a knock-out cell, a conditional knock-out or using knock-down of a specific protein involved in DNA break repair, which is described in more detail below.

Control samples, or cohorts thereof, can, e.g., be derived from subjects having a cancer, a specific grade of progression of a cancer, or an immunodeficiency such as common variable immunodeficiency (CVID) or a neurodevelopmental disease such as ataxia telangiectasia (AT).

It is of particular interest that the invention provides a method of predicting DNA repair efficiency in a subject, comprising carrying out the method of the invention. DNA repair efficiency in a cell that is a B-cell can be predicted, but as the mechanisms involved in DNA repair in the B-cells and in the body are basically the same, the method of the invention also allows for predicting DNA repair efficiency in a cell that is not a B-cell, such as a cancer cell, e.g., a cell of a solid cancer, for example, efficiency variations based on germline mutations.

As the method of the invention studies the outcome of DNA repair, it allows identifying distinct types of DNA-repair-related diseases. Most optimally, application of the method of the invention test early in life (as soon as the B-cell repertoire has gained sufficient diversity, e.g., after birth/neonatals, or, preferably, after about the age of 3) may be applied to predict an increased likelihood of the individual to develop cancer or whether it could suffer from an immunodeficiency. The inventor's data suggest that the method can guide treatment options for specific kinds of cancer, such as breast, ovarian, prostate, pancreatic cancer, melanoma, or lymphoma, e.g. B-cell lymphoma or T-cell lymphoma, e.g., whenever the cancer is associated with a germline or early somatic mutation.

More specifically, for example, it was shown that the method of the invention tool can discriminate breakpoint profiles of wild-type cell lines from those with a BRCA1 mutation with up to 89% confidence. Patients with BRCA1 or BRCA2 mutations may opt to have breasts removed because of breast cancer complications. Thus, the method of the invention can help to select a treatment option. An unfavorable outcome of the breakpoint profile analysis, i.e., a finding that a deficiency is present, could also be an indicator for continuous, life-long monitoring to ensure that cancers are diagnosed as early as possible so that broader treatment options are applicable.

Furthermore, the method of the invention may be helpful for adjusting treatment options that induce DNA damage (chemotherapy, radiotherapy, genome engineering). In individuals with poor DNA break repair, e.g., individuals that have a DNA break repair deficiency, DNA lesions are more likely to induce mutations that cause dangerous side effects. Chemotherapy is an umbrella term for the most commonly used drugs to treat cancer. They are chemicals whose selection for cancer treatment is based on the i) cancer type, ii) cancer stage and iii) historical response rate. The inclusion of an individual's capability to deal with DNA damage could be highly informative to prevent complications derived from the treatment. As the method of the invention studies the outcome of DNA repair, it allows identifying distinct types of DNA-repair-related diseases.

The present invention thus also provides a method for determining the risk to develop a cancer, an immunodeficiency such as common variable immunodeficiency (CVID) or a neurodevelopmental disease such as ataxia telangiectasia (AT).

Further provided is a method for determining the prognosis of a cancer, comprising carrying out the method of the invention.

The cancer can be, e.g., ovarian cancer, breast cancer, gastric cancer, lung cancer, melanoma, prostate cancer or glioma. The cancer can also be a lymphoma, e.g., a T-cell or B-cell lymphoma, but it is not limited to this.

The invention also provides a method for selecting a method of treatment of a disease selected from the group comprising a cancer, an immunodeficiency such as CVID or a neurodevelopmental disease such as AT, comprising carrying out the method of the invention, and selecting an appropriate treatment based on the DNA-break repair profile of the subject. For example, if the subject is found to have CVID, IVIg can be administered. If the subject has a neurodevelopmental disease, appropriate treatment thereof can be administered. If the subject is found to have a DNA break repair deficiency associated with a high risk of developing a cancer, or with a high risk of cancer progression, the subject can increase monitoring, e.g., decrease cancer monitoring intervals.

Identification of homologous recombination deficient (HRD, e.g., BRCA1/BRCA2 mutant) subjects, which can be provided by the method of the invention, has a high clinical importance, as, e.g., platinum, a very damaging chemotherapy, has an outstanding response in HRD+ ovarian cancer patients. This, if an ovarian cancer patient is found to be HRD, e.g., BRCA1/BRCA2 mutant, it is preferred that the patient is administered platinum.

However, it has been seen that HRD- ovarian cancer patients can also respond as well as HRD+ in some cases. Even though the balance between ovarian cancer remission to patient damage by platinum is so high, platinum treatment is presently tried in every woman to see if it works. Since HRD+ diagnostic often only considers BRCA mutations, some HRD- OC patients may carry mutations in the homologous recombination pathway. For instance, it has been recorded that BRCA WT patients with familial breast cancer had mutations in RAD51 gene. The method of the invention could indicate which ovarian cancer patient would be a good responder to platinum chemotherapy minimizing the risk of it not working.

The present invention also provides a computer program product comprising instructions which, when the program is executed by a computer, cause the computer to carry out analysis of step c) of the method of the invention. The computer-implemented program preferably comprises instructions to
a) filter reads, e.g., as explained herein,
b) cluster sequences to identify effective B-cell clones, e.g., as explained herein,
c) derive aggregated features from clustered read coverage, and
c) use machine learning to classify these features.

Optionally, at least 9 features, preferably, 10-20 or 15-20 features are used, most preferably, features selected from the group of features described above.

The invention also provides a kit suitable for carrying out the method of the invention, the kit comprising a computer-readable medium comprising the computer-implemented program of the invention and the primers suitable for carrying out the method of the invention, preferably, primers of SEQ ID NO: 1, 2 and 3, optionally, wherein at least one of the forward and the reverse primers further comprise a barcode sequence.

Throughout the invention, the term "about" is intended to mean +/- 10%. If not explicitly mentioned otherwise, "a" is to be understood as "one or more". All literature cited herein is herewith fully incorporated herein.

The invention is further illustrated by experimental examples and figures, which are not intended to limit the invention.

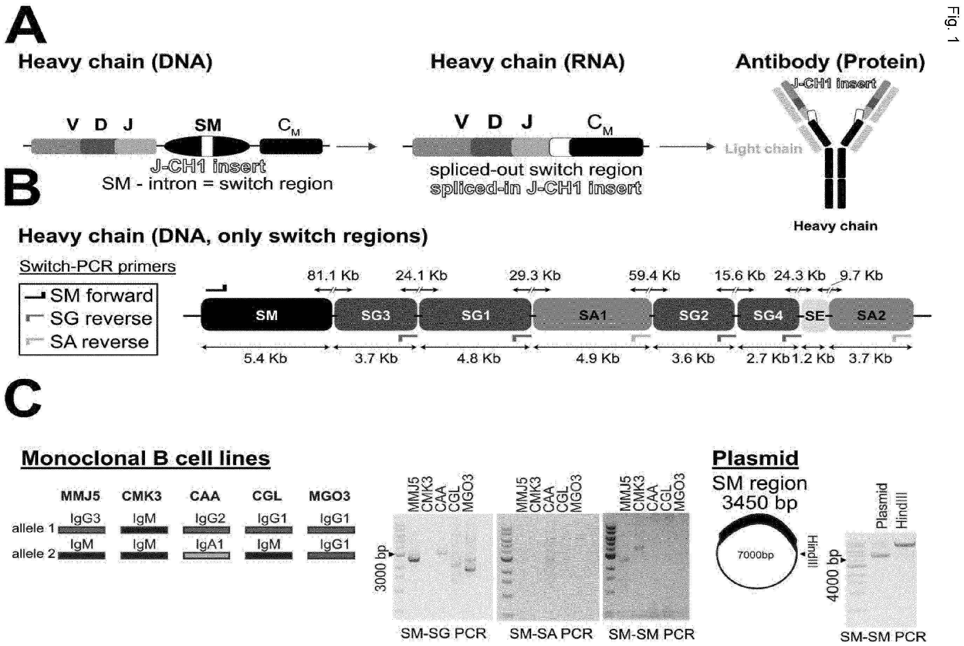

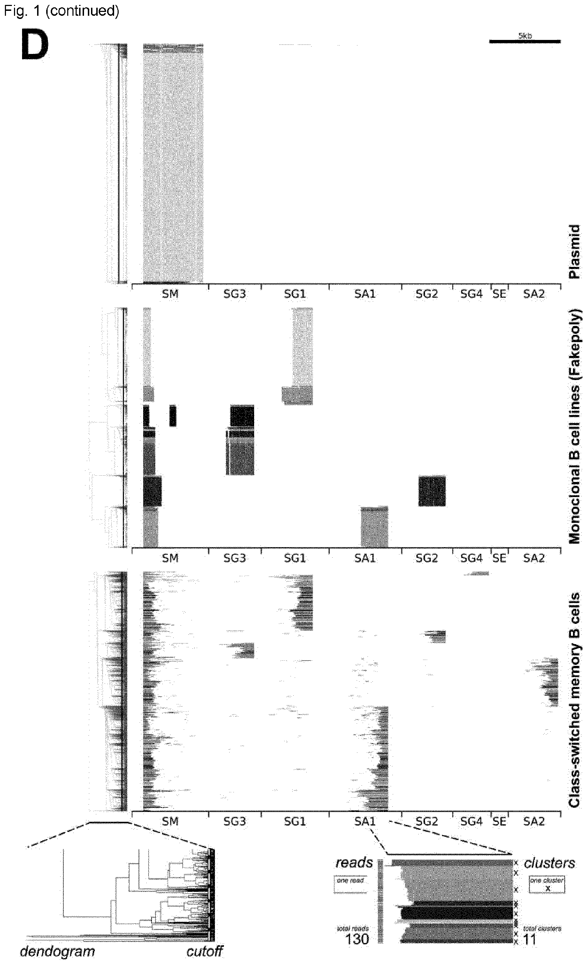

**Fig. 1** **SWIBRID: strategy and outcome of switch-joint analysis.**
   **A.** Depiction of the heavy chain gene, transcript and protein of a J-CH1 insert containing antibody. C_{M} = constant mu, SM = switch mu. J-CH1 insert, V,D,J domains and SM & C_{M}. **B**. Depiction of the switch regions of the human immunoglobulin heavy chain (IGH) locus indicating primer binding sites to amplify switch-joints. kb = kilobases. SM = switch mu; SG3 = switch gamma 3; SG1 = switch gamma 1; SA1 = switch alpha 1; SA2= switch alpha 2; SG2= switch gamma 2; SG4 = switch gamma 4 and SE = switch epsilon. Primers: SM forward (FW), SG reverse (RV) and SA RV. C. On the left side, the so-called "FakePoly" control comprises 6 switched alleles of monoclonal B-cells, deriving from EBV immortalized human B-cells, namely, MMJ5, CAA, CGL and MGO3 (CMK3 acts as a negative control because it does not carry a switched allele); a plasmid containing the human SM used as control after linearization using Hindlll. On the right side, 0.8% agarose gels show the switch-PCR amplification of the FakePoly or SM-SM PCR amplification conducted in the plasmid. 1 kb ladder was used. D. Read plots of samples with no, medium, and high B-cell diversity: plasmid < Fakepoly < class-switch memory B-cells. Five thousand random reads are depicted in each plot. On the left is a hierarchical clustering dendrogram of the switch-joint reads and the cut-off (white dashed line) used to determine the number of clusters that discriminates technical noise from biological diversity. Each straight line in the same horizontal plane represents an individual read or switch-joint amplicon. Reads depicted together and in the same shade belong to the same B-cell cluster. The reads appear to align the x-axis representing the IGH locus' switch regions.

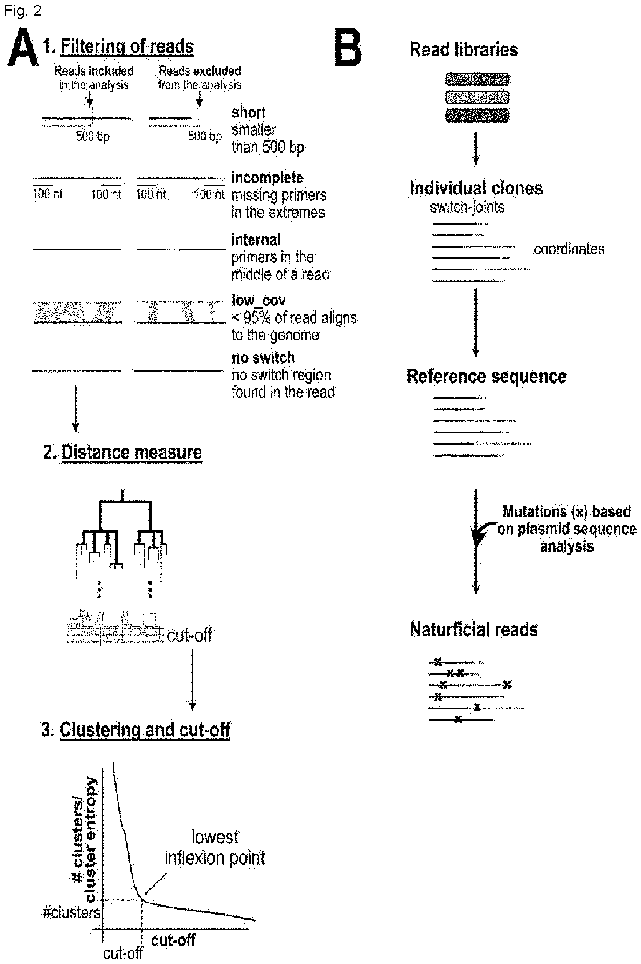

**Fig. 2** **SWIBRID functionality and development of templated synthetic switch-joint reads**
   **A.** Simplified scheme of SWIBRID's pipeline. First, a filtering process to exclude reads not fulfilling quality criteria is performed, followed by the measurement of read distances and last, the determination of cut-off, which takes part by controlling the maximum "cophenetic" (intra-cluster) distance. For instance from identifying an inflection point in a curve showing either the cluster number or the entropy of the cluster size distribution at different distance cut-offs..
   **B**. Scheme of naturally templated synthetic reads (naturficial read) generation. Different shades represent various switch regions. The black line represents SM.

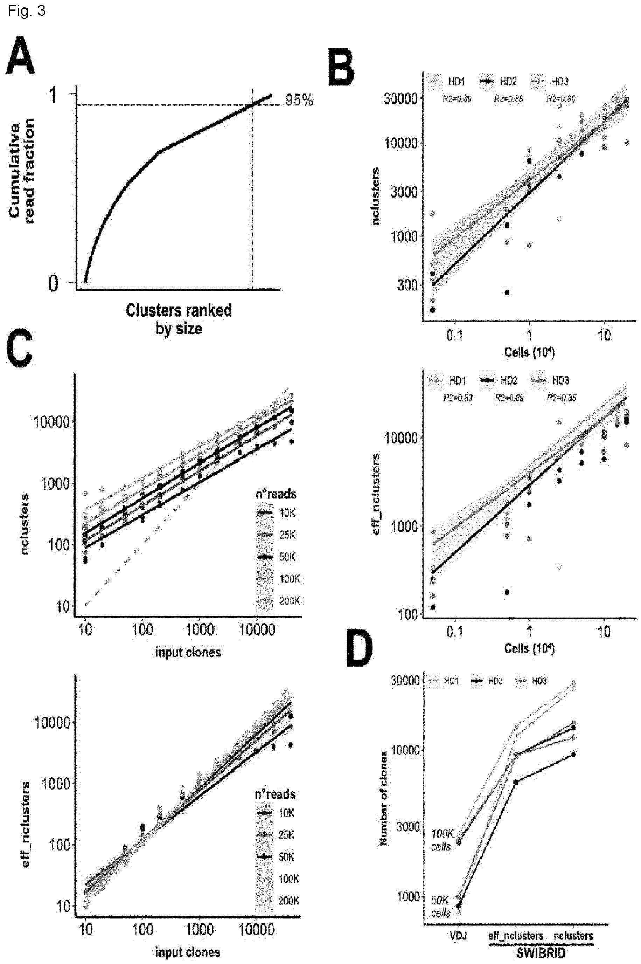

**Fig. 3****. Effective clusters identify individual B-cell clones after SWIBRID analysis of in vivo and in silico samples.**
   **A.** Explanatory scheme of identifying eff_nclusters identification using the cluster size and the accumulative number of reads. **B**. Human healthy donor samples run through SWIBRID. The input of IgG⁺/IgA⁺ B-cells is represented by the x-axis, while the output number, given by SWIBRID, of nclusters (top) and eff_nclusters (bottom), are represented on the y-axis. Three technical replicates were done for each cell number sample and donor. Lines represent the mean of the replicates per donor. HD1 and HD2 have three replicates per cell amount, HD3 has three replicates in 500, 5000, 25000, 150000, 200000, 100000 and, two replicates in 10000, 50000. **C.** Naturficial reads run through SWIBRID. The input clones are represented by the x-axis, while the output number, given SWIBRID, of nclusters (top) and eff_nclusters (bottom) are represented on the y-axis. The black line represents x=y. The letter "K" in the legend indicates thousand. **D.** Comparison of B-cell clones obtained by VDJ sequencing and SWIBRID using 50000 and 100000 memory IgGA+ B-cells of three donors. Points show the mean of three technical replicates.

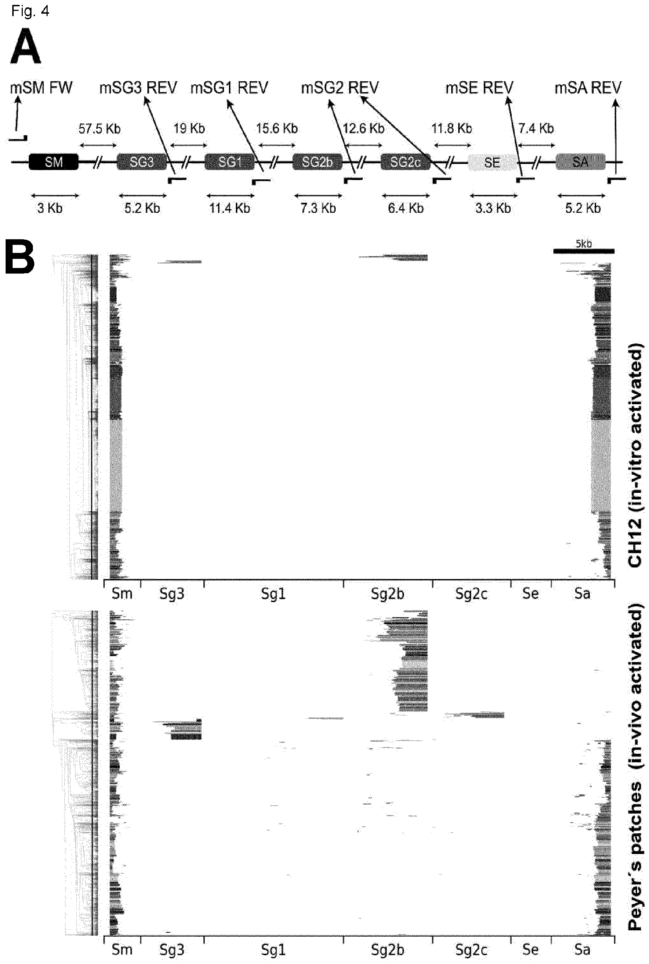

**Fig. 4** **SWIBRID in analysis of mouse samples**
   **A.** Switch regions of the mouse IGH locus show the primer binding sites. kb = kilobases. SM= switch mu; SG3 = switch gamma 3; SG1 = switch gamma 1; SA = switch alpha; SG2b= switch gamma 2b (6); SG2c = switch gamma 2c (6) and SE = switch epsilon. Switch region coordinates are indicated in Table M5. **B**. Readplots of *in vivo* and *in vitro* activated mouse cells (Peyer's patches and CH12, respectively). 5000 reads are shown in the plots. On the left is a hierarchical clustering dendrogram of the switch-joint reads and the cut-off (white dashed line crossing the dendrogram) used to determine the number of clusters. Each row represents an individual switch-joint read, and the x-axis represents the switch region's coordinates. Reads belonging to the same cluster are depicted in the same shade.

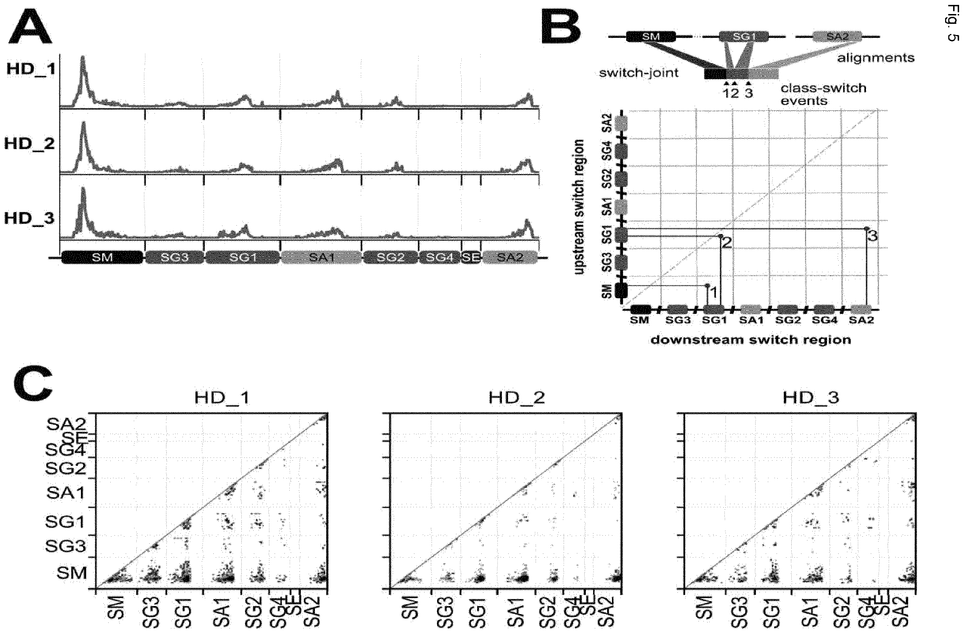

**Fig. 5** **A two-dimensional plot allows the depiction of the switch-joint breakpoints of healthy samples.**
   **A.** Breakpoint histograms from switch-joints of three healthy donors in 50 nt bins. Breakpoints are considered every break in the alignment of the switch-joint except primer binding sites. On the left is the sample annotation that corresponds to the histogram. Below are the annotations of the switch regions that align with the breakpoints. Grey boxes indicate the separation between the switch regions. **B**. Explanation of the two-dimensional breakpoint plot (2D-bps histogram). On the top is a scheme of a switch-joint containing sequences aligning to SM, SG1 and SA2. Triangles determine class-switch events. The lower scheme represents a 2D-bps plot with three dots corresponding to the number in the numbers next to the triangles. Dots are depicted considering the location of the breakpoint in the donor ("upstream"; y-axis) and acceptor switch region ("downstream"; x-axis). C. 2D-bps plot of healthy donors (HD) using 200 nt bins. These plots show the normalized (per eff_nclusters) density of breakpoints. Gradients indicate higher or lower breakpoints density.

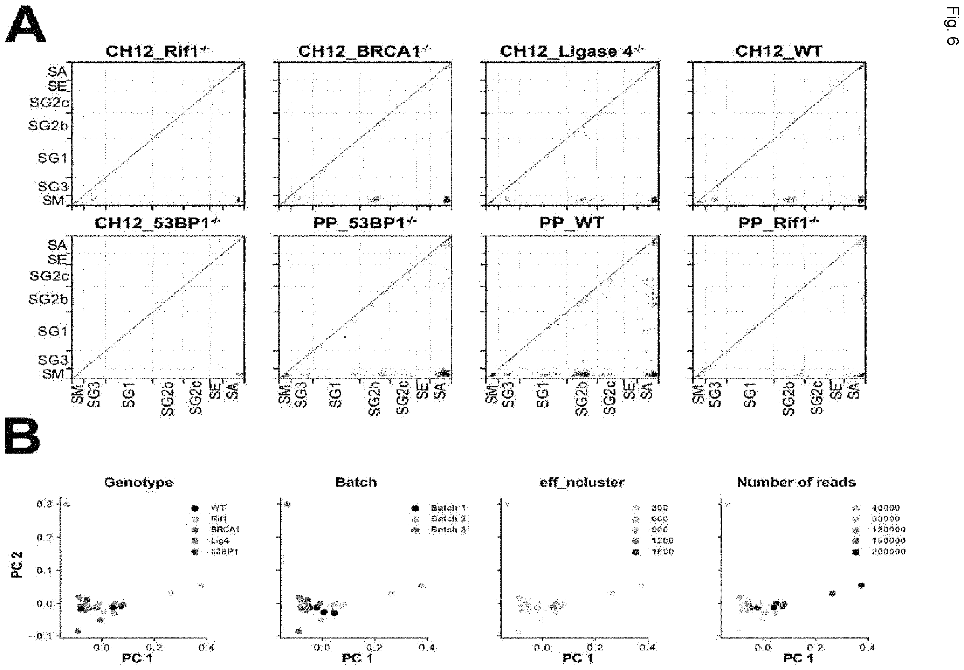

**Fig. 6** **Two-dimensional breakpoint plots based on murine samples**
   **A.** 6 2D-bps plots of standardized murine samples (by eff_nclusters) using a binsize of 200. CH12 = B-cell lymphoma cell line. PP = Peyer's patches. **B**. Principal component analysis (PCA) of CH12 samples carrying different knockouts (PC1 versus PC2). Knockouts are indicated in the graph in the "genotype" plot. WT = wild-type. Legends of the plots are located in the lower part of the PCA plots. The title of the plot indicates what the coding of the legend shows. CH12 WT, n = 6; CH12 Rif1^{-/-}, n = 7; CH12 BRCA1^{-/-}, n = 6; CH12 Ligase 4^{-/-}, n = 6; CH12 53BP1^{-/-}, n = 4.

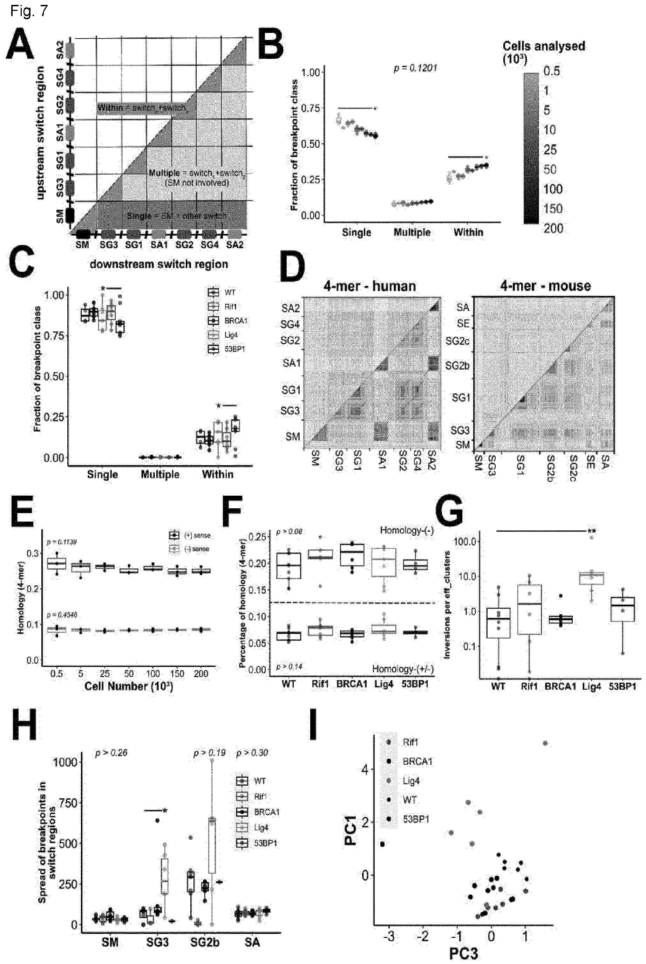

**Fig. 7** **DNA repair protein deficiencies show a different inversion frequency but no alteration in breakpoint class distribution.**
   **A.** Scheme of breakpoint classification using the 2D-bps histogram. Areas described as single, multiple, and within are depicted in the 2D-bps plot, respectively. **B**. Breakpoint class distribution along a different sampling of the HD4. Three technical replicates were performed for each cell number analyzed. C. Difference of multiple, single, and within breakpoint frequency between DNA repair knockouts and wild-type CH12 samples. **D.** Scheme of the 4-mer homology density between switch regions along the IGH. For every homology 4-mer nucleotide, a dark square was depicted. The lower triangle depicts the homology occurring between (-)-sense sequences in the IGH, while the upper triangle depicts the homology occurring between (+)- and (-)-sense sequences in the IGH. 4-mer means the homology analysis has been done between bins of 4 nucleotides. On the left, human, on the right, mouse. **E.** Percentage of homology used in switching among different cell number samples of HD4. **F.** Difference of average sequence homology used among different DNA repair protein knockouts in CH12 between (-)-sense sequences (homology-(-), upper) and between (+)- and (-)-sense sequences (homology-(+/-), lower). **G.** Inversions per eff_nclusters among DNA repair protein knockouts in CH12. **H.** Spread of breakpoints in SM, SG3, SG2b and SA among DNA repair protein knockouts in CH12. Spread indicates the average width of the switch regions covered by a sample **I.** PCA of DNA repair protein knockouts and wild-type CH12 samples. PCA was performed using scaled log10 inversions per eff_nclusters, single and within breakpoints, and spread in SG3 and SG2b. WT, n = 10; Rif1, n = 8; 53BP1, n = 5; BRCA1, n = 6 and Ligase 4, n = 7. T-test was performed after the normal distribution of the data was confirmed through the Shapiro test. * = p-value < 0.05.
**Fig. 8** **Representation of the accuracy of genotype identification as a function of the features used in a ranking from most to less important.**
   (upper left) - Identification of the genotypes without batch correction. (upper right) -Identification of WT or disease without batch correction. (lower left) - Identification of the genotypes with batch correction. (lower right) - Identification of WT or disease with batch correction. Y-axis = accuracy, x-axis= # of features. LR=logistic regression, SVC=support vector classifier, RF=random forest.

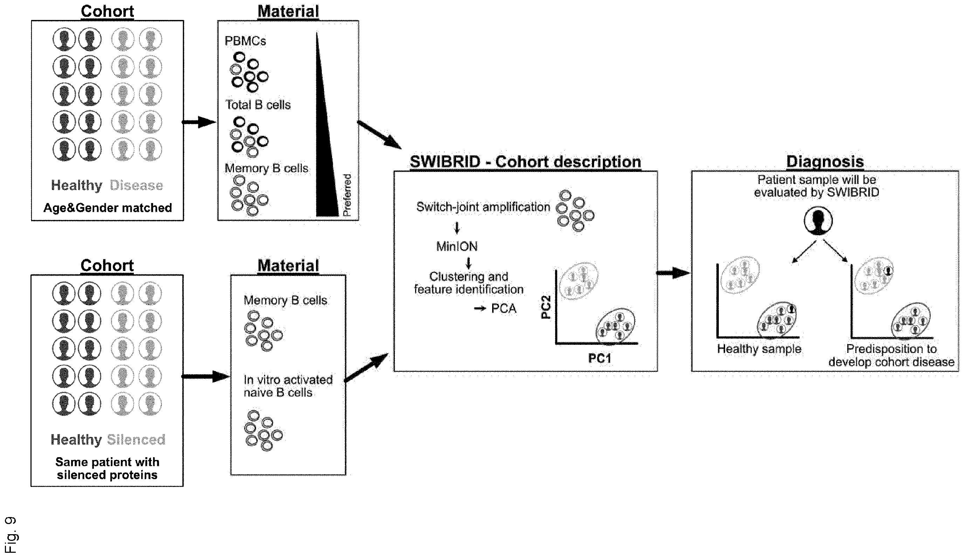

**Fig. 9** **DNA repair biomarker scheme**
   Cohort patients in black represent the healthy controls, and patients in grey represent the diseased or silenced donors. Cells represented in grey stand for cells of interest. PCA = principal component analysis. Instead of PCA, machine learning can be used.

### Examples

### Material and Methods

### Primary human B-cell culture

Primary human B-cells were cultured using 10% FBS (PAN Biotech GMBH), 1% non-essential aminoacids (Life Technologies), 1% sodium pyruvate (Life Technologies), 1% GlutaMAX (Life Technologies), 1% Penicilin-Streptomycin (Life Technologies), 0.1% mercaptoethanol (Life Technologies), 5 pg/ml Kanamycin (Serva Electrophoresis,) and 0.002 % Transferrin (Merck-Milipore) 1M HEPES RPMI (Life Technologies).

Primary human B-cells were cultured in a 12-wells plate (SARSTEDT) at 200.000 cells in 1 ml with 4 Gy irradiated CD40L expressing K562L cells to induce their activation. The culture was carried out for seven days. On days 0, 1, 3, 5, 25 ng IL4 (recombinantly produced) was added to each well to trigger activation. If the primary human B-cells were treated with some chemicals, they were added simultaneously with the IL4. Cells were harvested on day 7. gDNA isolation of primary human B-cells was performed using the ReliaPrep^{™} Blood gDNA Miniprep System (Promega).

### CH12 cell culture

CH12 cells were cultured using 10% FBS (PAN Biotech GMBH), 1% non-essential aminoacids (Life Technologies), 1% sodium pyruvate (Life Technologies), 1% GlutaMAX (Life Technologies), 1% Penicilin-Streptomycin (Life Technologies), 0.1% mercaptoethanol (Life Technologies), 5 µg/ml Kanamycin (Serva Electrophoresis) and 0.002 % Transferrin (Merck-Milipore) 1M HEPES RPMI (Life Technologies).

CH12 were cultured in a 12-wells plate (SARSTEDT) at 200.000 cells in 1 ml. The culture was carried out for three days. On day 1, 25 ng IL4 (recombinantly produced), 4300 ng of anti-mouse CD40 (BIOZOL) and 8.6 ng of mTGF-b1 (R&D Systems) were added to each well to trigger B-cell activation. Cells were harvested on day 3. gDNA isolation of CH12 was performed using the ReliaPrep^{™} Blood gDNA Miniprep System (Promega).

### Flow cytometry & fluorescence-activated cell sorting (FACS)

The preparation of cells for flow cytometry and Fluorescence-activated cell sorting (FACS) was carried out in the same fashion. The only difference was the number of cells used and the device where the staining was performed. Flow cytometry staining was performed in a 96-well plate U-bottom (SARSTEDT) with 25 µl of staining solution and 50.000 to 200.000 cells. FACS staining was performed in a 15 ml falcon tube (Faust) with 800 µl of staining solution and 15 to 50 million cells.

The staining was carried out by centrifuging the cells at 500g for 5 minutes. Then, cells were resuspended in the staining solution that contained the antibodies chosen for the analysis and incubated for 7 minutes at 4°C in the dark. Cells were rinsed with eight times more volume of MACS buffer and centrifuged for 5 minutes at 500g. Cells were washed with DAPI solution, rinsed with eight times more MACS buffer and centrifuged for 5 minutes at 500g. Cells for flow cytometry were resuspended in 400 µl of MACS buffer and cells for FACS were resuspended in 500 to 1000 µl of MACS buffer, depending on the number of stained cells.

Flow cytometry was done in LSRFortessa^{™} Cell Analyzer (BD Biosciences) and FACS was done in BD FACSAria^{™} Fusion Flow Cytometers (BD Biosciences). First, single staining of the colors used was used to calculate compensation. Then 50.000 events were recorded from each sample. Analysis was done using FlowJo (BD Biosciences).

| **Target** | **Fluorophore** | **Info** | **Dilution** | **Analysis** |
|---|---|---|---|---|
| CD27 | PE | Miltenyi Biotec, #130-114-156, M-T271 | 32 | FACS |
| IgD | PE-Cy7 | Miltenyi Biotec, #130-098-584, IgD26 | 32 | |
| IgM | AF488 | Life Technologies, #A21215 | 160 | |
| IqG | AF647 | Dianova, #109-606-170 | 80 | |
| IqA | AF647 | Dianova, #109-606-011 | 80 | |
| DAPI | UV | BD Biosciences, #564907 | 2000 | |
| IqM | AF488 | Life Technologies, #A21215 | 100 | Flow cytometry |
| IqG | AF647 | Dianova, #109-606-170 | 500 | |
| IqA | AF647 | Dianova, #109-606-011 | 500 | |
| DAPI | UV | BD Biosciences, #564907 | 2000 | |
| Table M1. Antibodies used in flow cytometry and FACS for different analyses. | | | | |
| The staining solution was done using MACS buffer. Alexa Fluor = AF | | | | |

### Generation of artificial switch-joint reads

Templated artificial (naturficial) reads are described as naturally templated computationally generated reads. The reference sequences of 10000 unique B-cell clones from 8 HD were obtained. Samples comprising a different amount of clones and reads were made. The naturficial reads were mutated randomly following the MinION^{®} mutations observed in the non-PCR MinION^{®} control:

| | **A** | **C** | **G** | **T** |
|---|---|---|---|---|
| **A** | **0.190063** | 0.000193921 | 0.0116397 | 0.000554028 |
| **C** | 0.000574434 | **0.292938** | 0.000341318 | 0.00207396 |
| **G** | 0.0112465 | 2.17643e-05 | **0.288772** | 0.000121508 |
| **T** | 0.001054 | 0.00121019 | 8.39998e-05 | **0.199111** |
| Table M2. Mutation rates between nucleotides to generate naturficial reads. | | | | |
| Mutations were obtained from a sample run in MinION^{®} without PCR (the SM plasmid). | | | | |

These mutations comprised single nucleotide mutations (Table M3) and indels (chance to get an insertion = 0.0200868 with an increasing size probability of 0.511982 and a deletion = 0.0253724 with an increasing size probability of 0.687357).

### Primary human B-cell isolation from blood

Buffy coats were purchased from the DRK-Blutspendedienst Nord-Ost GmbH. All buffy coats were free from antibodies against HIV, HCV and HBV. Informed consents were provided by all donors and samples were fully anonymized.

Firstly, 80 ml of blood was mixed with 20 ml of 2 mM EDTA PBS-T (Promega & Biotek). The blood was distributed in 4 falcons, slowly pouring 30 ml of the blood on top of 15 ml of Ficoll (Carl Roth GmbH). Falcons are centrifuged for 25 min at 500g with an acceleration/break of 3/0 (Centrifuge Eppendorf 5910R). Blood was separated density-wise and lymphocytes were isolated from the white layer between the plasma and the Ficoll. The isolated lymphocytes were washed twice with 2 mM EDTA PBS-T.

Primary human B-cells were isolated using CD19 Microbeads (Miltenyi Biotech). 200 µl of CD19 Microbeads were incubated in ice with 500 million lymphocytes for 15 minutes in 800 µl. Then, they were washed using 3 ml MACS buffer (10% FBS 2 mM EDTA PBS-T) and centrifuged. Lymphocytes were resuspended in 3 ml MACS buffer to make them pass through a 30 µm pre-separation filter (Miltenyi Biotech) and an LS Column (Miltenyi Biotech) attached to a QuadroMACS separator (Miltenyi Biotech). LS Columns were washed three times using 3 ml of MACS buffer. Then, 5 ml of MACS buffer was used for flushing the CD19-positive lymphocytes with the help of the syringe provided by the LS Columns.

### MinION^{®} library preparation

PCR products and amplicons were purified using ProNex beads (Promega) in a 1:1 ratio following manufacturer protocol. Amplicons were eluted in 16 µl.

Purified switch-joint amplicons were firstly quality controlled. Thus, 5 µl of purified amplicons were checked in a 0.8% Agarose (Biozym) gel. In addition, amplicons were loaded into 2100 Bioanalyzer (Agilent) using 1 µl of it. Amplicons visible in the gel were diluted 50% before loading them into 2100 Bioanalyzer.

The outcomes of both assays were used to decide how much volume is added from each sample. Sample loading was done according to manufacturer guidelines.

The total volume of DNA amplicons should be 48 µl. If the volume was less, it was corrected using the elution buffer to elute the amplicons. The MinION^{®} library preparation (MinION^{®} Nanopore, #SQL-LSK109) was followed as indicated in the protocol from the manufacturer, except, DNA control was not used when preparing the MinION^{®} library. Flowcells were suitable for runs when they had more than 800 pores available.

### Switch-joint PCR

| | **Coordinates** | **Sequence** | **Chromosome** | **Type** | **Orientation** | **Assembly** |
|---|---|---|---|---|---|---|
| SM | 105860975-105861001 | CACCCTTGAAAGTA GCCCATGCCTTCC (SEQ ID NO: 1) | 14 | Primer | forward | GRCh38 |
| | 106327185 - 106327211 | | | | | GRCh37 |
| SM | 105856877-105856902 | GGAACGCAGTGTAG ACTCAGCTGAGG (SEQ ID NO: 5) | 14 | Primer | reverse | GRCh38 |
| | 106322982 - 106323007 | | | | | GRCh37 |
| SA | 105588857-105588879 | CTCAGTCCAACACC CACCACTCC (SEQ ID NO: 2) | 14 | Primer | reverse | GRCh38 |
| | 105709106-105709128 | | | | | |
| | 106055194 - 106055216 | | | | | GRCh37 |
| | 106175443 - 106175465 | | | | | |
| SG | 105626824 - 105626852 | | 14 | Primer | reverse | GRCh38 |
| | 105645549 - 105645577 | | | | | |
| | 105743825-105743853 | | | | | |
| | 105772166-105772194 | | | | | |
| | 106093161 - 106093189 | | | | | GRCh37 |
| | 106111886 - 106111914 | | | | | |
| | 106210162 - 106210190 | | | | | |
| | 106238503-106238531 | | | | | |
| SE | 105601669 - 105601692 | | 14 | Primer | reverse | GRCh38 |
| | 106068006 - 106068029 | | | | | GRCh37 |
| SM | 113426178 - 113426200 | GGAGGGACCCAGGC -    TAAGAAGGC (SEQ ID NO: 6) | 12 | Primer | forward | GRCm38 |
| | 113389798-113389820 | | | | | GRCm39 |
| SA | 113260804 - 113260830 | GCAAGCAGTGGACC CAAAGACGAGAGG (SEQ ID NO: 7) | 12 | Primer | reverse | GRCm38 |
| | 113224424 - 113224450 | | | | | GRCm39 |
| SG 1 | 113330330-113330354 | | 12 | Primer | reverse | GRCm38 |
| | 113293950 - 113293974 | | | | | GRCm39 |
| SG 3 | 113361218 - 113361244 | | 12 | Primer | reverse | GRCm38 |
| | 113324838 - 113324864 | | | | | GRCm39 |
| SG 2 | 113307913-113307936 | CTGATGGGGTGTT GTTTTGGCTG (SEQ ID NO: 8) | 12 | Primer | reverse | GRCm38 |
| | 113288916-113288936 | | | | | |
| | 113271533 - 113271556 | | | | | |
| | 113252536 - 113252556 | | | | | |
| SE | 113273349-113273369 | | 12 | Primer | reverse | GRCm38 |
| | 113236969 - 113236989 | | | | | GRCm39 |
| Table M3. Primers that can be used for switch region PCR in human (GRCh38 / GRCh39) and mouse (GRCm38 / GRCm39) antibody locus. | | | | | | |

### Human switch-joint PCR

Human switch PCR starts by mixing the different reagents as suggested for the manufacturer protocol of LongAmpTaq^{®} (NEB). The reaction is done in 25 µl and the optimal template amount is the isolated gDNA of i) 25.000 memory B-cells or ii) 200.000 PBMCs. In one reaction, the forward primer Sµ is added in combination with Sα and Sγ reverse primers (Table M4). The PCR protocol comprises the following steps: 95°C for 3 min and 25 cycles of 95°C for 40s, 60°C for 30s, 65°C for 3 min; and the last elongation step at 65°C for 10 min. Different temperatures are reached on a ramp of 4°C/s.

### Mouse switch-joint PCR

Mouse switch PCR starts by mixing the different reagents as suggested for the manufacturer protocol of LongAmpTaq^{®} (NEB). The reaction is done in 25 µl and the optimal template amount is 100-150 ng of isolated gDNA. In one reaction, the forward primer Sµ is added in combination with Sα and Sy2bc reverse primers and in another combination Sµ in combination with Sy3 and Sε reverse primers (Table M4). The PCR protocol comprises the following steps: 95°C for 3 min and 30 cycles of 95°C for 40s, 60°C for 30s, 65°C for 3 min; and the last elongation step at 65°C for 10 min. Different temperatures are reached on a ramp of 2°C/s.

### Statistics

Tests were two-tailed, and a p-value lower than 0.05 was considered statistically significant. Shapiro-Wilk test was used for normality testing of continuous variables, using R function shapiro. test ( ). An independent Student t-test was used when continuous data met the criteria for normality, using the R function t. test ( ) ; otherwise, the Mann-Whitney U test was used, using the R function wilcox.test (). Principal component analysis (PCA) was performed with scaled data and under the same seed using R (seed = 147). PCA was performed using the R function prcomp::prcomp ( ).

### SWIBRID

| **Switch region** | **Specie** | **Chromosome** | **Coordinates** | **Assembly** |
|---|---|---|---|---|
| SM | Human | 14 | 106322600-106328000 | GRCh37 |
| SG3 | Human | 14 | 106238000-106241700 | GRCh37 |
| SG1 | Human | 14 | 106209000-106213800 | GRCh37 |
| SA1 | Human | 14 | 106174500-106179600 | GRCh37 |
| SG2 | Human | 14 | 106111400-106115000 | GRCh37 |
| SG4 | Human | 14 | 106093000-106095700 | GRCh37 |
| SE | Human | 14 | 106068712-106069622 | GRCh37 |
| SA2 | Human | 14 | 106055000-106058700 | GRCh37 |
| SM | Mouse | 12 | 113423700-113426700 | GRCm38 |
| SG3 | Mouse | 12 | 113361000-113366200 | GRCm38 |
| SG1 | Mouse | 12 | 113330500-113341900 | GRCm38 |
| SG2b | Mouse | 12 | 113307500-113314800 | GRCm38 |
| SG2c | Mouse | 12 | 113288400-113294800 | GRCm38 |
| Table M4. Switch region consideration to align for SWIBRID | | | | |

Then, the reads that do not meet quality criteria are discarded. Quality criteria are the following:
- Reads are longer than 500 nt
- Reads contain the SM FW primers and the SG REV or SA REV within the 100 nt beginning and end of the read
- Reads should not contain single primers outside the 100 nt of the beginning and end of a read
- The sequence of the read must contain at least one part of a switch region, as defined herein (Table M4)
- The sequence of the read must align in more than 95% of its length to the genome

If more than 50 nt of the sequence does not align to switch regions, it will be considered a J-CH1 insert as long as its sequence aligns in 95% of its length to the genome and it is flanked by switch regions.

If two primers are found together in the middle of the read, the read should be split and considered as two independent reads.

Reads that pass the filter are aligned to the genome and transformed into a coverage pattern. The coverage pattern is used for clustering. The clustering follows a hierarchical agglomerative clustering, using a distance metric based on the clean coverage pattern. The hierarchical agglomerative clustering was made using the Cosine distance. The distance measure will develop a dendrogram later used for the cut-off determination.

The cut-off limits the differences allowed to take part in cluster determination. In order to determine the cut-off, the function of clusters and cut-off is used. In this function, two tendencies are observed, i) one with a high slope observed at low cut-offs, and ii) one with a lower slope observed at larger cut-offs. The middle point between these two slopes is used to determine the cut-off and thus, the number of clusters.

### RESULTS

### Analysis of switchjoints to decipher DNA repair well-function in humans and mice

DNA repair malfunction predisposes people to develop diseases such as cancer. DNA repair malfunction is often addressed by studying the specific DNA repair proteins, given that researching somatic DNA repair is complex due to the low frequency and randomness of somatic DNA breaks in cells. The inventors decided to focus on researching DNA repair function by studying somatic breaks in the antibody locus during B-cell diversification. One of these events is CSR, a controlled deletional recombination process in the antibody locus that joins two distant switch regions (switch-joint) to give rise to a new isotype. The switch-joints result from a DNA repair event that commonly carries mutations and insertions and can be analyzed as a DNA repair footprint.

Further, non-immunoglobulin elements deriving from distant genomic regions integrate into the heavy chain antibody locus (IGH), thereby adding another layer of diversity to the antibody repertoire (8, 9). More specifically, insertions in switch regions (J-CH1 inserts) can be spliced into antibody transcripts and consequently incorporated as extra elements in the antibody protein. The mechanism of J-CH1 insert acquisition is still unclear. Recent studies identified J-CH1 inserts in antibody transcripts with a mean length of 160 bp. In rare cases, inserts were estimated to comprise up to 10 kb in the genome when counting their multiple exons separated by large introns. The study was performed using suppression PCR to enrich for long antibody transcripts. This semiquantitative approach does not allow for an definite quantification of inserts per clone (3). An unbiased approach to identify J-CH1 inserts would allow the quantification of B-cell clones and, therefore, the J-CH1 insert frequency (J-CH1 insert per B-cell clone). As RNA is analyzed, suppression PCR is only suitable to detect successfully spliced J-CH1 inserts while genomic inserts lacking splice recognition sites will not be detected. The latter would however be important when studying the J-CH1 insert mechanism. In other words, the J-CH1 insert mechanism should be studied at the DNA level using a tool to identify the insertions and unique B-cell clones for quantitative analysis (Fig. 1A).

The switch-joints have been previously studied using short sequencing (Illumina^{®} or Ion Torrent^{®}), which captured only a few nucleotides covering the switch-joints (10-13). However, the inventive method aims to study a bigger picture of the switch-joints using L-NGS MinION^{®} and screen CSR breakpoints, sequential switching, intra-switch deletions and J-CH1 inserts in large stretches of switch-joints.

Thus, the invention aimed to develop a tool to analyze switch-joints and characterize their variabilities (e.g., J-CH1 inserts and breakpoints) within a library, determining unique B-cell clones using long amplicons, e.g., derived from MinION^{®}.

### Development of a bioinformatical tool to identify breakpoint profiles, unique B-cell clones and J-CH1 inserts using long reads:

### Development of a tool that overcomes MinION^{®} errors in the sequence and identifies individual B-cell clones in a sample:

Building upon previous work (8), the inventors first aimed at improving the methodology that integrates i) amplification of switch-joints comprising the switch µ and switch γ 1, γ 2, γ 3, γ 4, α 1 or α 2 (SM, SG1, SG2, SG3, SG4, SA1, SA2) (8), ii) sequencing by MinION^{®} technology and iii) computational analysis capable of quantifying B-cell clones via switch-joint analysis and J-CH1 inserts. The primers were designed to amplify only switch-joints, e.g., SM-to-SG3, while entire switch regions that did not undergo CSR were excluded from amplification, because the smallest distance found between the forward primer and any reverse primer used in an IGH locus not subjected to class-switching is 81.1 kb (SM-SG3) (Fig. 1B), which is far beyond any PCR amplification limit (14).

Switch-joint amplification (e.g., switch-PCR) generates long amplicons with a mean length of 2.5 kb. Long amplicons can be sequenced using L-NGS devices, e.g., MinION^{®} or PacBio^{®}, or by Illumina^{®} upon fragmentation of amplicons. The latter generates highly accurate reads with a maximum read length of 2x300 bp. However, short reads demand reconstitution and assembly of amplicons using computational tools. Considering that switch regions are characterized by long, repetitive GC-rich sequences, accurate reconstitution of fragmented switch-joint amplicons is excluded. Thus, the inventors decided to sequence their amplicons using an L-NGS device, PacBio or MinION^{®} (Nanopore technology^{®}). PacBio gives rise to more accurate reads than MinION^{®} (error rate <1% versus 5%, respectively), but it is also known to give rise to a lesser read yield. MinION^{®} is a more flexible technology than PacBio, due to the possibility of sequencing without the need for PCR amplification. MinION^{®} is also a technology that has rapidly evolved. The inventors decided to use MinION^{®} despite its 5% error rate, which was tackled bioinformatically.

In a previous study, J-CH1 insert frequency was standardized using total reads, which in turn meant that every read was considered as one B-cell clone (8), which likely led to wrong assumptions about J-CH1 insert frequency because, in a polyclonal B-cell library, unique B-cell clones are represented by several reads. Therefore, the invention provides a tool to identify individual B-cell clones, e.g., for a proper J-CH1 inserts frequency quantification. To this end, the inventors developed a bioinformatical tool called **SWIBRID (SWitch region Breakpoint Repertoire IDentification),** the **first computational tool to identify unique B-cell clones using switch-joints** that can be used in the method of the invention.

SWIBRID discards reads that do not meet quality criteria (e.g., as recited herein), can identify breakpoints of switch-joints, J-CH1 inserts and isotypes, classify the switch-joint amplicons in individual B-cell clones, and characterize the switch-joint B-cell clones, by determining the number of reads that represent them.

Establishing controls to ensure SWIBRID performance in B-cell clustering by switch-joint analysis was helpful. SWIBRID read input contains errors due to PCR amplification and MinION^{®} sequencing, which can add technical noise to the reads library and potentially lead to the overestimation of clones. Therefore, two control samples were generated to control for i) PCR amplification by amplifying switch-joints of well-defined B-cell monoclonals pooled together that we called "FakePoly" (Fig. 1C); and ii) MinION^{®} by sequencing a linearized plasmid containing the SM region using MinION^{®} (Fig. 1C).

To visualize the output of SWIBRID comprehensively, the readplot was developed. The readplot visually shows the clustering of a library for a fraction of randomly chosen reads (Fig. 1D). The readplot represents the read's alignment to the IGH locus, using the x-axis to reference the switch regions. For this purpose, the x-axis depicts the SM, SGs, SE and SAs regions in the same order found in the IGH locus. Exons and intergenic regions of the IGH locus were excluded from the x-axis. The y-axis indicates the individual reads. Every read starts in SM, due to the usage of SM FW primer, at the very left side of the plot and ends in a gamma or alpha region. Consequently, sharp edges can be found at the beginning of SM and the end of every other switch region. Reads are represented by fine, straight lines depicted from left to right and are only visible over the switch region parts to what they align. Reads considered to belong to the same B-cell clone are depicted together and in the same color; thus, the diversity of a B-cell sample is represented by the variety of colors in a readplot.

The read plots serve as a clustering visualization tool to judge the sample's diversity. Visual comparison of read plots comprising 5000 reads of plasmid (low diversity), FakePoly (medium diversity) and class-switched memory B-cells (high diversity) show clear differences. Plasmid portrays a unique big cluster, FakePoly, a few big clusters and the class-switched memory B-cells several small clusters (Fig. 1D).

In summary, the inventors identified individual clusters from a switch-joint read library produced by MinION^{®} using SWIBRID. Such analysis provided the readplots, a clustering visualization tool that allows for the sample's diversity judgment.

### Towards the development of SWIBRID: the tool to characterize switch-joints:

The success in clustering polyclonal B-cell samples using switch-joints further let to development of SWIBRID. SWIBRID comprises i) filtering the reads that do not match our quality criteria, ii) optionally, identifying J-CH1 inserts, iii) measuring the differences between reads and iv) determining the number of individual B-cell clones in a sample (Fig. 2A).

**MinION^{®} reads were first filtered to obtain a high-quality library to be clustered.** The quality criteria involved discarding: reads shorter than 500 bp, considering that an extended range sequence analysis was desired (short); reads not containing primers at both ends of the reads, hypothesizing that the sequencing of the read has been abrogated during the MinION^{®} run (incomplete) and the reads in which less than 95% of the sequence map to the genome (low_cov) (Fig. 2A). Filtering MinION^{®} reads is also important because PCR amplification can create artifacts that could generate false diversity. Indeed several artifacts were observed by manual analysis. Secondly, the manual analysis identified the so-called "no-switch" reads. These reads do not align to the switch regions in any part of its sequence. Considering that switch-joints were of interest, it was decided to discard these reads. Nevertheless, differentiation between no-switch reads and reads containing part of switch regions plus other parts of the genome was needed, as the latter could be a J-CH1 insert-containing read. Following previous steps in identifying J-CH1 inserts in switch-joints (8), the inventors decided to identify J-CH1 inserts as a sequence belonging to a read of at least 50 nt long flanked by switch regions and which sequence aligns at least 95% to the genome. Manual analysis of the J-CH1 insert-containing read confirmed the accuracy of SWIBRID identification. In summary, the filtering step discarded short, incomplete, low_cov, internal and no-switch reads and identified J-CH1 inserts in parallel.

**The distance measure computes the differences between reads.** To quantify similarities and differences between reads, the alignment blocks were converted into binary coverage patterns in the switch region, i.e. matrices of dimensions (# reads x # positions), where a value of 1 means coverage at a position and 0 means no coverage. Optionally, coverage in the antisense direction could be encoded by negative values. Gaps in coverage smaller than 75nt were removed. These coverage patterns (the rows of the matrix) were then clustered using Jaccard or Cosine distance and hierarchical clustering, visualized by the dendrogram shown in Fig. 2A.

The next step is to identify the number of individual B-cell clones. In order to do so, a **cut-off is determined.** The cut-off sets the limit of differences allowed to identify unique clusters (considered B-cell clones). To visualize the diversity of samples, the inventors plotted the number of clusters or cluster entropy (y-axis) obtained at varying cut-offs (x-axis). Of note, two clear tendencies were observed. For very small cut-offs, the graph showed a steep slope representative of the technical noise, and beyond a certain cut-off, another tendency could be shown with a lower slope representative of the biological diversity (Fig. 2A). A reasonable cut-off could be determined at the lowest point of the inflection, or by usage of the x coordinate of the inflection point in the curve. The clusters obtained at the calculated cut-off were called nclusters.

SWIBRID aims to represent B-cell diversity through switch-joints by only considering events that would appear in nature. In order to achieve this, SWIBRID follows the following B-cell class-switch biology rules:
- A switch-joint can include more than two different switch regions. B-cells can class-switch more than one time, due to a process called sequential switching, and
- Class-switching can only occur following the (-) sense direction of the IGH locus due to the intragenetical deletional recombination. In other words, if the SM-SG1 joint occurs, SG3 should not appear after SG1 (Fig. 1B), or it would be considered a J-CH1 insert (15).
- Further optimizations were introduced. One significant problem experienced was the case of an extensive set of reads that did not contain a barcode. Twenty reads from the undetermined reads were manually analyzed and identified as the control DNA used by the library preparation SQK-LSK109 Kit (Nanopore technology^{®}). Since then, the control DNA has not been used in the library preparation and the libraries were more enriched in the sample reads.
- In summary, SWIBRID"s structure follows highly optimized biological and computational rules, making it possible to identify J-CH1 inserts and individual B-cell clones using switch-joint sequencing reads generated, e.g., by MinION^{®}.

### Analysis of naturficial reads and human polyclonal B-cell samples show that the read numbers do not impact SWIBRID output in highly diverse samples:

When comparing read numbers and the number of B-cell clones identified by SWIBRID, the inventors hypothesized that the number of reads could impact the number of B-cell clones detected in a sample: fewer reads, fewer clones. If that were the case, comparing samples with different amounts of reads could bias results. To address this problem, two measures were taken: i) generation of artificial switch-joints representing different amounts of clones at distinct reads and ii) analysis of different cell numbers in triplicates and different healthy donors and observation of the impact of the technical variabilities at different diversities, considering that one of the variabilities is the number of reads obtained from a run.

*In silico* reads were created computationally. This approach generated natural templated *in silico* clones based on the coordinates of previously identified B-cell clones by SWIBRID, ensuring the natural distribution of isotype switching and breakpoint acquisition which we called naturficial reads. In detail, B-cell clones were produced using the reference sequence of nclusters found in 8 samples from 4 donors and later mutating them with the corresponding degree and quality of mutations that the SM plasmid control (Table M2), which is not amplified by PCR, acquired after a MinION^{®} run (Fig.2B). The minimum and the maximum number of individual naturficial B-cell clones generated were 10 and 10000, respectively. Naturficial reads are considered an *in silico* library.

Secondly and to analyze human samples, three healthy donors (HD) were analyzed in triplicates using different cell numbers to test SWIBRID reproducibility considering technical variabilities (read number, PCR amplification, MinION^{®} flowcell sequencing etc.). This experiment will be referred to as the cell number experiment. Primary human B-cells were isolated, and memory IgG and IgA (CD27⁺IgG⁺/IgA⁺) were sorted, gating CD27⁺IgG⁺/IgA⁺ in total B-cells, e.g., from HD1: 10.8%, HD2: 14.5% and HD3: 6.5%. Based on the quality between the input and output in a PCR reaction, 500, 5000, 10000, 25000, 50000, 100000, 150000 and 200000 memory IgG and IgA B-cells were identified as the most suitable samples to be tested. A linear relationship between the input cells and the SWIBRID output B-cell clones was expected. PCR reactions using these cell numbers were performed in three technical replicates for each donor. The isolation of gDNA of the three healthy donors and the switch-joint PCR was performed following the same protocol. Before sequencing, the switch-joint PCR result was checked by gel and Bioanalyzer (data not shown).

SWIBRID analysis of the cell number experiment and naturficial reads comprised identifying unique switch-joint B-cell clones using two units: nclusters and eff_nclusters. **nclusters are defined by the total amount of unique switch-joint B-cell clones found in a sample at a defined cut-off** (Fig. 2A). **eff_nclusters is defined by the smallest number of clusters that contain 95% of reads** (Fig. 3A). Considering that the analysis is based on B-cell biology and that MinION^{®} has a 5% error rate, the inventors decided to ignore small nclusters and look at the data in a more realistic way giving rise to eff_nclusters. This approach was chosen following the VDJ-sequencing B-cell repertoire analysis, which discards reads or clusters under specific quality rules (17). These two units are essential to differentiate since nclusters contain singletons (clusters that only have one read), and nclusters included in eff_nclusters generally contain at least 15 reads (value obtained from the average amount of minimum reads in the clones of eff_nclusters in human samples).

SWIBRID analysis of the cell number experiment and naturficial read libraries identified an amount of B-cell clones that increased with the input number of cells or clones, respectively. There was almost no difference between nclusters and eff_nclusters in the three healthy donors of the cell number experiment, as shown by the correlation index, R2. In both cases, healthy donors showed a linear tendency between input cells and SWIBRID switch-joint B-cell clones (Fig. 3B). However, the maximum amount of nclusters and eff_nclusters found were 27000 and 26000, respectively, even though the input amount of cells for these values was 100000. The naturficial reads only used a maximum amount of input clones of 10000, which, in principle, and based on the cell number experiment, SWIBRID should identify efficiently. eff_nclusters identified in naturficial reads followed an intersected line (dashed line in Fig. 3C) that showed an equal number of SWIBRID and input clones, which means that SWIBRID could successfully identify the exact amount of input clusters (Fig. 3C-bottom). On the contrary, the tendency of nclusters diverged from the intersected line at low and high number of input clones (Fig. 3C-top). The difference in the values obtained of nclusters and eff_nclusters in naturficial reads could be due to the impact of the MinION^{®} mutations in the clustering. MinION^{®} mutations could falsely create unique switch-joint B-cell clones that would not cluster together with other reads, creating singletons. Therefore, the usage of eff_nclusters is preferred over the nclusters. Thus, SWIBIRID can identify B-cell clones successfully using eff_nclusters and, optimally, the maximum eff_nclusters identified should not be higher than 20000.

The samples of the cell number experiment gave rise to different amounts of reads. Fearing that the number of reads would have affected SWIBRID output, the inventors analyzed the different input clones of the naturficial reads using 10000, 25000, 50000, 100000 and 200000 reads. nclusters, but not eff_nclusters, identified in naturficial reads increased with the number of reads used in the sample (Fig. 3C). The results supported the usage of eff_nclusters over nclusters since it was not subjected to the number of reads.

SWIBRID results showed that the three healthy donors did not elicit more than 27000 unique switch-joint B-cell clones even though the input cell triplicated that value (Fig. 3B). Since the method was novel, it was decided to gain further insights to understand if SWIBRID was underestimating the clonality of samples. Currently, the only method to analyze B-cell clonality is the VDJ-sequencing. VDJ-sequencing identifies unique B-cell clones based on the combination of V-D-J-constant domains (VDJ-clone) (18). Considering that the saturation of SWIBRID B-cell clone identification begins at 50000 cells, we ran VDJ-sequencing in the three healthy donors using 50000 and 100000 cells as inputs. VDJ-sequencing revealed that the three healthy donors had a maximum of 1000 and 3000 VDJ-clones for 50000 and 100000 input cells, respectively (Fig. 3D). Therefore, SWIBRID might not be underestimating the B-cell clones identified. The vast discrepancy between the unique B-cell clones found in the VDJ-sequencing and SWIBRID can be explained by the fact that they identify different types of clones. While VDJ-sequencing differentiates B-cell clones generating antibodies binding to different antigens, SWIBRID differentiates between B-cell clones that have undergone different CSR rounds. Thus, one VDJ-clone can be covered by different SWIBRID clones.

In summary, naturficial reads showed that by identifying the eff_nclusters, the additional diversification caused by MinION^{®} mutations was avoided. Also, it was successfully confirmed that the number of reads comprising a sample does not play a role in eff_nclusters identification. In addition, the fact that there was not such a high difference between the number of nclusters and eff_nclusters in the cell number experiment showed that the add-on mutations by MinION^{®} did not affect the analysis. Finally, VDJ-clones are not equal to SWIBRID eff_nclusters since they represent diversification with regards to antigen specificity and class switching, respectively.

### Translation to murine B-cells: in vivo and in vitro models of mouse switch-joint diversity:

After successfully generating a workflow to analyze human samples using SWIBRID, an analysis of J-CH1 insert incorporation by modulating DNA repair proteins *in vitro* was of interest. Nevertheless, the *in vivo* analysis of samples carrying DNA repair protein deficiencies is also important for understanding the impact of DNA repair proteins in the natural J-CH1 acquisition. Human DNA repair protein deficiencies are rare diseases, implying that procuring such material is limited. In fact, research on DNA repair protein deficiencies is done more often in mice than in humans. Therefore, translation to the murine system would increase the research possibilities.

CH12 is a mouse lymphoma B-cell line that almost exclusively switches to IgA (19). WT and CRISPR/Cas9 knockout CH12 (Replication timing regulator 1 (Rif1)^{-/-}, Ligase 4^{-/-}, 53BP1^{-/-} and BRCA1^{-/-}) were activated *in vitro* using IL4, mTGF-b1 and anti-CD40L. Also, primary mouse B-cells were isolated from Peye"s patches (PP) of WT mouse intestines that mainly switch to IgA *in vivo* (*20*)*.*

The mouse switch-joint PCR was designed following the human workflow, which only allowed the amplification of switch regions in IGH loci subjected to class-switching. Primers were designed based on the murine switch region annotations (Table X1 ) and, in the case of mSM FW primer, based on Shinkura et al., 2004 (21) (Fig. 4A). Switch-joint PCR worked for all the designed primers *in vitro* and *in vivo* activated mouse B-cells; however, when the primers carried the 20 nt barcodes, mouse SM-SG1 and SM-SE failed to be amplified (data not shown). As barcodes allowed for multiplexing, and numerous attempts to make the amplification of mouse SM-SG1 and SM-SE failed, the analysis of the IGH locus was limited to SM-SG3, SM-SG2b.c and SM-SA switch-joints, considering the materials used from mice, PP and CH12, contained mainly SM-SA switch-joints. Unlike the human barcoded switch PCR, the mouse PCR was done using barcoded mSM FW and the unbarcoded SA/SG2b.c/SG3 RV primer. 1.1-fold diversity was gained using one barcoded primer over two barcoded primers. The translation to murine samples in the SWIBRID pipeline involved the usage of the genome assembly GRCm38 and the annotations of the murine switch regions (see above).

SWIBRID was tested using WT *in vivo* and *in vitro* activated mouse samples. The *in vivo* activated sample, PP, showed 395 eff_nclusters, of which 4.3 % were SG3, 30.3 % were SG2b, 3.5 % were SG2c, and 59.7 % were SA. On the other hand, the *in vitro* activated sample, CH12, showed 417 eff_nclusters, of which 90.4 % were SA. Most clones were derived from SM-SA class-switching, which is expected due to the nature of PP and CH12. Regarding diversity, the four most prominent clusters in CH12 occupied 47.3% of all the reads, while in PP, only 6.9%. This result showed that PP generates higher diverse switch-joints than CH12 (Fig. 4B).

In conclusion, the method was successfully translated to mouse samples. A lower isotype diversity was found in mouse samples, which is expected due to the nature of the cell line CH12, and the non-immunized mouse from which PP were obtained.

### Providing a DNA repair biomarker:

SWIBRID was originally developed to study J-CH1 inserts; however, the efficiency of J-CH1 insert identification is very low. According to the data, J-CH1 inserts were found in 10 per 1000 reads, leading to the unused portion of 990 reads per 1000 reads. However, SWIBRID analysis not only recorded data from J-CH1 inserts but also profiled the breakpoints occurring in the switch-joints of the samples. Switch-joints carry the scars of the dsDNA breaks that have occurred during CSR. Since the switch-joints and any other somatic dsDNA break are most likely repaired by cNHEJ or aEJ, one can consider the switch-joint breakpoint profile to reflect dsDNA break repair in the whole body. Therefore, the inventors considered that analysis of the reads obtained by SWIBRID could help to understand somatic dsDNA break repair in healthy and sick patients.

Current clinical tools do not look at the genomic scar left by DNA repair to understand DNA repair efficiency in patients but rather at mutations driving DNA repair malfunction. Developing a tool that identifies healthy and DNA repair deficient samples based on the switch-joint breakpoint profiles could prognose diseases related to B-cell biology or DNA repair mechanisms with the advantage that DNA repair players that have not been yet identified would not be missed. Thus, the inventors decided to apply the method they had developed as the basis of a DNA repair biomarker.

### Two-dimensional breakpoint plots reveal specific breakpoint patterns in human samples:

Firstly, the switch-joint analysis started by finding a way of visualizing the data. SWIBRID breakpoint analysis of healthy donors was depicted in a histogram that showed the breakpoints accumulating in the core of the switch regions. Breakpoint histograms depicted every class-switching event as two independent events, one in the donor switch region (upstream) and one in the acceptor switch region (downstream). Three healthy donors (HD) switch-joint breakpoints were depicted in the histogram. There was no apparent difference except for HD3 that had an abnormal peak in distribution in SG1. However, all three HDs shared the position of the peaks among the switch regions (Fig. 5A).

The class-switch event results from one repair event in which the DNA repair machinery joins two switch regions. It is important to consider the sequence features of the parts that had to be joined to pinpoint specific repairs carried out by certain proteins, e.g., RAD51 would use high stretches of homology to repair the DNA. Consequently, a two-dimensional breakpoint histogram (2D-bps histogram) was developed that counts every switching event as one considering the exact position of the switch regions involved. The 2D-bps histogram has a y-axis representing the donor switch regions and an x-axis representing the acceptor switch regions. The 2D-bps histogram depicts every switching event using the coordinates of the acceptor and donor switch region (Fig. 5B, Table X2). The 2D-bps histogram does not consider every nucleotide as a single position but instead uses binning to depict and analyze the data. A bin size is determined by grouping nucleotides as units. For instance, if a bin size of 500 is chosen, the sequence would be divided into groups of 500 nucleotides. This affects the analysis, as any nucleotide present in a group can be considered a match in the bin. The 2D-bps histogram allowed for the observation of direct switch events between SM and other switch regions, intra-switch deletions and class-switch events between two switch regions that are not SM as a result of sequential switching.

Switch-joint breakpoints of HD were depicted in the novel 2D-bps histogram. This depiction allowed us to observed a more detailed landscape of CSR processes that we were not able to observe before using the histogram (Fig. 5C). For instance, HD2 looked very similar to HD1 in the histogram, however, HD2 seems to have less diverse switch-joint breakpoints in the middle of the plot when compared to HD1. The inventors decided to analyze the switch-joint breakpoints using the 2D-bps plot using CH12 knockouts of DNA repair proteins to see if there are difference in break repair between the WT and the sick genotypes.

In summary, the method of the invention can analyze healthy samples, e.g., by depicting class-switching events in a two-dimensional plot of two axes representing the donor and acceptor switch regions and independent from the switch-joint isotypes of the samples. Small differences between HDs drove the inventors to try the tool in CH12 knockouts of DNA repair proteins.

### Application of 2D-bps histograms to classify murine DNA repair deficient samples:

Human B-cells contain a high diversity in their switch-joints since humans are constantly exposed to pathogens pushing B-cell diversification to generate highly variable antibodies. The variability accounts for the switched isotype and the randomness of the breakpoints throughout the switch regions. Since the human IGH contains 8 isotypes and is ~200 kb long, the variability among switch-joints can be immensely high. Thus, defining a healthy DNA break repair profile is challenging, and a large cohort should be used to analyze breakpoint profiles in switch-joints. Optimally, DNA repair deficient human samples would be used to establish the DNA repair biomarker in humans, but such humans are scarce. Hence, DNA repair biomarkers were determined in mice because CH12 knockouts are available. Considering that it is a cell line, several replicates of the different CH12 knockouts were generated and analyzed using SWIBRID to estimate if the switch-joints breakpoint profiles could separate the different knockouts.

In contrast to polyclonal human samples, activation of CH12 generated a lower number of eff_nclusters. Firstly, because they mostly switch to SA, and secondly, one potential explanation is that since they are a B-cell line with a common ancestor, they might class-switch using a pattern. WT PP samples were also analyzed to compare the diversity found in CH12 to an actual mouse. Due to the lower number of eff_nclusters, the visualization of the 2D-bps plots became less apparent to the eye. Hence, a binsize 200 was used for visualization, while the analysis of the 2D-bps plot was done using a binsize 50 to comply the analysis done in human samples. Visualization of CH12 and PP using 2D-bps plots showed a clear difference in IGH locus usage. Keeping in mind that the switch-joints analyzed in mouse samples were SM-SG3, SM-SG2b, SM-SG2c and SM-SA, CH12 mainly showed breakpoints focused on the SM-SA area, while PP showed a higher diversity of breakpoints along the whole IGH locus with a higher frequency in SM-SA. The comparison of CH12 2D-bps plots shows that WT and BRCA1^{-/-} have very similar breakpoint profiles in SM-SA and SM-SG2b, although CH12 WT has higher breakpoints in the latter. CH12 Ligase 4^{-/-} also had a majority of breakpoints in the SM-SA zone but was also scarce in the SM-SG2b zone. CH12 53BP1^{-/-} and Rif1^{-/-} has almost no breakpoints; most were focused on the SM-SA zone (Fig. 6A).

PCA based on 2D-bps histograms of CH12 samples standardized by eff_ncluster and a binsize of 50 did not reveal a separation between the CH12 knockouts. In an attempt to study the core problem of the lack of separation between the knockouts and the wild-type samples, the genotypes (WT, Rif1^{-/-}, BRCA1^{-/-}, Ligase 4^{-/-} and 53BP1^{-/-}), the MinION^{®} run date (batch 1, batch 2, batch 3), the eff_nclusters and the number of reads in each sample (nreads) were visualized in the PCA plot (PC1 versus PC2) (Fig. 6B). Comparison between these features showed that the date of the MinION^{®} run had the highest impact since different batches appeared clustered independently of the CH12 genotype (Fig 6B2).

In conclusion, analysis of the 2D-bps histograms using CH12 knockouts allowed us to use a higher number of replicates per genotype and revealed limitations in the capacity of the PCA analysis of 2D-bps plots to differentiate between samples. The inventors thus considered that development of a DNA repair biomarker would be further enhanced by deconvolution of the 2D-bps histograms into single characteristics.

### Exploiting the 2D-bps histograms in human and mouse samples-- the DNA repair biomarker:

2D-bps histograms can classify the breakpoints at different levels: single breakpoints, breakpoints occurring between SM and any other switch region, multiple breakpoints, breakpoints occurring between two switch regions that are not SM (as a result of sequential switching) and within breakpoints, breakpoints that occur within the same switch region (Fig. 7A). Classification of breakpoints is potentially crucial to separate DNA repair deficient from healthy donors because DNA breaks in DNA repair deficient patients are more likely to be left unrepaired, which poses a threat to the life of the B-cells. Thus, DNA repair deficient B-cells would likely not carry as many breaks as healthy samples because the ones with breaks would likely die.

Analysis of breakpoint classes in different cell number samples from HD4 (Fig. 7B) showed that there was an inversional difference between the highest and lowest number of cells in single and within breakpoints, unlike in multiple breakpoints that there were no differences. This difference can be explained by the fact that this unit is represented by the fraction of breakpoints and not the number. There is a higher chance of finding the same clone within a population when the sample is bigger. Quantification of breakpoint classification standarised by eff_nclusters means that only one event per eff_ncluster would count and thus, in samples of smaller cell amount a higher diversity distribution among its reads will be found than in samples with higher number of cells. Nonetheless, no explanation for the increase of within breakpoints as a function of the number of cells was found. On the other hand, CH12 WT, 53BP1^{-/-}, BRCA1^{-/-}, Ligase 4^{-/-} and Rif1^{-/-} were cultured for two days in two rounds, generating 10, 5, 6, 7 and 8 replicates, respectively. SM-SA, SM-SG3 and SM-SG2bc switch-joints were amplified, MinION^{®} sequenced and ran in SWIBRID. Comparison of breakpoint classification in CH12 showed a general lack of multiple breakpoints, or sequential switching, and a significant difference in single and within breakpoint frequency between WT and BRCA1^{-/-} CH12 samples (Fig. 7C).

Furthermore, DNA repair protein deficiencies trigger the replacement of preferred DNA repair mechanisms. The preferred DNA repair mechanisms during CSR are c-NHEJ and a-EJ, which often use <20 nt homologies. Homologous repair (HR) or single-strand annealing (SSA), which work exclusively using >20 nt homologies, can take over the repair of dsDNA breaks when cNHEJ or aEJ cannot be used. Since such a different homology range is found in homology and non-homology DNA repair mechanisms, the average sequence homology used for repair in switch-joints was analyzed.

Homology was calculated by measuring how often both regions joined by a break shared the same small sequence motif. Instead of comparing the strands nt by nt, the analysis was done for 4 nt motifs; thus, it was called **homology 4-mer.** In this analysis, raw sequences were not directly compared since MinION^{®} errors could lead to false results. As an alternative and trusting SWIBRID's alignment of the libraries to the IGH, the homology 4-mer was calculated using the coordinates of the reads, specifically, the 2D-bps plot. First, we depicted the homology of the reference sequence in 4 bins in a plot with the same configuration (x- and y-axis) as the 2D-bps histogram (Fig. 5B). The homology plot showed the most homologous sequences in black and the least in white. The homology 4-mer in samples comprises the average homology of 50-bin breaks, i.e., by summing up the homology of all bins weighted by the frequency of breaks in each bin. Furthermore, the analysis differentiated between the homology 4-mer occurring in two strands following the same sense, homology (-) 4-mer (Fig. 7D-lower triangle), and two strands of opposite sense, homology (+/-) 4-mer (Fig. 7D-upper triangle). The differentiation was necessary to consider the occurrence of inversions during CSR. Results show that the average sequence homology (-) or (+/-) 4-mer used by HD4 at different cell numbers was maintained (Fig. 7E). Following the same lines, CH12 samples did not show a different average sequence homology (-) or (+/-) 4-mer (Fig. 7F).

With the purpose of finding specific features to separate WT from CH12 knockout cells, the inventors decided to record the number of inversions occurring in a sample per eff_ncluster. Inversions are CSR events in which the join happens between a (-)-sense switch region and a (+)-sense switch region. Ligase 4^{-/-} CH12 had a significantly higher inversion frequency than any other CH12 sample (mean inversion per eff_nclusters: Ligase 4 = 25.8, BRCA1 = 0.92, Rif1 = 2.81, 53BP1 = 2.06, WT = 1.3) (Fig. 7G). These results agree with the fact that 53BP1, a core protein in the DNA repair machinery during CSR working together with ATM and Ligase 4, has been found to regulate inversional joins tightly.

Due to the lack of apparent differences among all the different knockout samples, a new feature was added to the analysis, the spread in the switch regions. The spread represents the average width of the switch regions covered by breakpoint samples. The aim was only to add the spread switch regions that would add differences. Spread in SM and SA was very similar among all samples; however, spread in SG3 showed significant differences between WT and Ligase 4^{-/-} samples and SG2b showed different tendencies (Fig. 7H).

The analysis could not consistently separate CH12 knockouts from WT using the previous features separately. Therefore, the inventors decided to perform principal component analysis (PCA) using the scaled breakpoint classes, inversion frequency, homology (-) 4-mer and homology (+/-) 4-mer. PCA is a linear multi-variable analysis that calculates how distant samples are from each other based on the variables given (PC) and ranks the PCs from the highest to the lowest in terms of their ability to account for the variance between samples (PC1, PC2, PC3 ...). Visual separation of samples based on two different PCs would indicate that samples differ based on the variables analyzed. The PCA was done using the variables that showed a higher difference (significantly or not) among the samples: single breakpoints, within breakpoints, inversion per eff_nclusters, spread in SG3 and SG2b. Depiction of PC1 and PC2 clustered the different mutants together. Ligase 4^{-/-} samples clustered separated at a certain distance from the other samples, and 53BP1^{-/-} and Rif1^{-/-} clustered together. Clustering of 53BP1^{-/-} and Rif1^{-/-} together might signify that similar switch-joints result from a knockout of proteins that promote c-NHEJ only when working together (Fig. 7I).

In summary, using features derived from clustered read coverage patterns that can be visualized in some cases using the 2D-bps histogram, in CH12 helps clustering the knockouts and the WT samples separately using a PCA. The clustering of knockouts allows for using the method of the invention as a DNA repair biomarker.

### Ranking of features to identify CH12 genotypes

SWIBRID analyzes the breakpoint profiles of switch-joints and characterizes them using more than 90 different features. In an attempt to use SWIBRID more effectively, the inventors decided to pinpoint the most important features to identify the CH12 genotypes and avoid comparing 90 features between the samples.

The ranking was used to determine the number of features necessary to differentiate the genotypes using machine learning. Three different machine learning were used i) logistic regression (LR), ii) support vector classifier (SVC) and iii) random forest (RF). Estimations indicated that the optimal number of features to identify the genotypes is between 15 and 20 when using them in the order of the ranking (Figure 8 left). Also, similar results were obtained when the identification was focused on determining the sample as WT or disease (Figure 8 right). The highest accuracy was observed when using LR (Figure 8).

In conclusion, the analysis shows that the optimal way to identify the CH12 genotypes is by using less than 20 features. SWIBRID usually uses logistic regression for the identification of the genotypes.

### identification of CH12 genotypes using machine learning

SWIBRID was built to identify genotypes from samples. Therefore, the inventors predicted the genotype of blinded samples using SWIBRID, including 11 DNA repair deficient samples.

Firstly, the inventors ran 46 CH12 samples including 10 WT, 11 Ligase 4^{-/-}, 9 Rif1^{-/-}, 9 BRCA1^{-/-} and 7 53BP1^{-/-}. These 46 samples were used to train machine learning. Then, the blinded samples were run in MinION^{®}. SWIBRID features were used to predict their genotype using the trained machine learning. Surprisingly, 9 out of 11 DNA repair deficient samples samples were correctly identified:

**Table M5. Blinded identification of CH12 genotypes**

| **predicted genotype** | **Rif1 #1** | **Rif1 #2** | **Rif1 #3** | **Lig4 #1** | **Lig4 #2** | **Lig4 #3** | **BRCA1 #1** | **BRCA1 #2** | **53BP1 #1** |
|---|---|---|---|---|---|---|---|---|---|
| **53BP1** | 0.57 | 0.15 | 0.17 | 0.02 | 0 | 0 | 0.04 | 0 | 0.3 |
| **BRCA1** | 0.06 | 0.03 | 0.35 | 0.02 | 0.01 | 0.02 | 0.39 | 0.89 | 0.03 |
| **Lig4** | 0 | 0 | 0 | 0.94 | 0.96 | 0.96 | 0.06 | 0 | 0.22 |
| **Rif1** | 0.11 | 0.8 | 0.48 | 0 | 0 | 0 | 0.2 | 0.09 | 0.14 |
| **WT** | 0.25 | 0.02 | 0 | 0.01 | 0.02 | 0.02 | 0.31 | 0.01 | 0.31 |
| **match** | | | | | | | | | |

The top row lists the CH12 knockouts that SWIBRID analyzed. The left column indicates the genotypes predicted by machine learning. Values indicate confidence in the prediction. The lower row indicates successful (white) or failed (black) predictions. Machine learning was trained using 46 samples, excluding the samples applied in this experiment.

In conclusion, SWIBRID features are sufficient to identify distinct CH12 genotypes using machine learning. Thus, SWIBRID features can identify potentially threatening human genotypes that may result in cancer, and the method can be used as a diagnostic tool.

### DISCUSSION:

### B-cell switch-joints represent a biomarker of DNA repair deficiencies:

### SWIBRID analysis can be extrapolated to the DNA repair in the genome and finds mutations more frequently than current methods:

The present invention provides SWIBRID, a novel tool to identify DNA repair deficiencies by analyzing the breakpoints occurring in switch-joints of B-cells. The analysis comprises the characterization of breakpoint profiles in the switch-joints. It was found to be advantageous to comprehensively characterise breakpoint profiles by means of a limited number of derived features for the identification of DNA repair deficiencies.

In SWIBRID, the mutational break repair of CSR, processed by cNHEJ, aEJ and SSA was studied rather than the error-free break repair. By definition, the latter results in a non-mutational DNA repair, meaning that no indel or single nucletide polymorphism (SNP) would be recorded after the well-functioning of HR (23). In fact, DNA repair by HR can leave the IGH locus as if it never broke, impeding the switch-PCR amplification. On the other hand, the mutational break repair results in mutations, in the case of CSR, a deletion in the form of a switch-join in the IGH locus, allowing a PCR spanning the switch regions to work.

Unrepaired DNA breaks trigger apoptosis, which suggests that the cell intrinsically seeks to repair a DNA break instead of allowing it to remain broken. Indeed, if a DNA repair pathway misses a protein, it will try to compensate for it using other proteins, potentially repairing a DNA break into a threatening outcome. For instance, the deficiency of an HR protein would lead to a higher rate of mutations since the DNA repair would be processed more often by a mutational DNA repair pathway. Considering that switch-joints are a sink of somatic DNA breaks, they were analyzed using SWIBRID to identify DNA repair deficiencies. The analysis was specifically able to separate WT, BRCA1^{-/-}, Ligase 4^{-/-}, 53BP1^{-/-} and Rif1^{-/-} CH12 knockouts using PCA and including the features: single and within breakpoints, inversion per eff_nclusters, spread in SG3 and SG2b. In humans, mutations in BRCA1, Ligase 4 and 53BP1 are linked to the development of cancer.

The malfunction of DNA repair has been commonly researched, screening cancer-driving mutations because DNA repair malfunction is highly related to the development of cancer(24). Specifically, Sherman et al., 2022 analyzed public whole-genome, whole-exome and targeting sequencing datasets of 37 cancer types using a deep learning approach called Dig. Dig generated a neural network that could identify positively selected cancer-driving mutations of arbitrary cancer cohorts (25). Dig allowed the identification of new cancer-driving mutations linked to DNA repair function. For instance, E74 Like ETS Transcription Factor 3 (ELF3), a transcription factor involved in HR (26), is an oncogene whose malignancy has been previously linked to its gene variants (27). Nonetheless, Dig newly found mutations in the ELF3 promoter in two cancer cohorts. The mutations allowed ELF3 methylation potentially linked to an overexpression (25). Cancer-driving mutations are crucial to identify, screen for, and asses people's predisposition to cancer. However, screening all cancer-driving mutations found in Sherman et al., 2022 would involve the analysis of at least a whole-exome sequencing, which is expensive to carry out for everyone. Most importantly, analysis of DNA repair protein sequences would not certainly link mutations with malfunctions, it would only find DNA repair protein variants, however, SWIBRID would be able to spot DNA repair malfunctions. Therefore, the approach of SWIBRID to analyze DNA repair function and, indirectly, cancer predisposition, using the breakpoint profile of switch-joints, could be used in the clinics as a predisposition analysis.

The inventors analyzed the switch-joints from B-cells, assuming that switch-joints mirror the somatic DNA repair in the body. DNA repair research considers a successful CSR in B-cell proof that cNHEJ or aEJ works. Findings such as the role of 53BP1 for end-joining DNA repair or the inactive Ligase 4 role in ligating DNA breaks are based on the fact that a B-cell was able to class-switch and their switch-joints were screened. Most importantly, it has been shown that the types of mutations happening during B-cell diversification are the same that trigger B-cell lymphoma, linking somatic DNA repair to cancer. Specifically, Machado et al., 2022 analyzed the mutational landscape of B-cells and T-cells in different subpopulations and compared the mutational burden found in whole-genome sequencing in four healthy donors to their progenitor cells. They found that in lymphoid malignancies SBS9, mutations in A:T base pairs in a TpW context, are frequently found in the genome, and the memory B-cells carrying such mutations show enriched off-target SHM, C:G mutations, in highly expressed genes (28). This fact links the mutations found in the IGH to the mutations in the whole genome of the cell. More importantly, it has been found that neurons and oligodendrocytes accumulate mutations with age and follow the same mutational burden as the healthy donor progenitor cells that Machado et al., 2022 analyzed (29). Since neurons and progenitor cells, two completely different cell types, similarly mutate their genome, we can suggest that switch-joints in B-cells do the same and could mirror the DNA repair throughout the body.

These studies were unbiased since they did not focus on specific genomic regions and could analyze either predisposition to cancer or mutational burden using whole-genome sequencing. However, the disadvantage of using such a methodology is the low frequency of finding a mutation. Sherman et al., 2022 used publicly available sequencing data from cancer cohorts. They looked for mutations using 10 kb-bins, allowing them to find a mutation per 1.3 bins analyzed (~ 13 kb). In contrast, Machado et al., 2022 sequenced the whole genome using 3000 cells per sample. The samples consisted of an expansion of individual clones belonging to different cell subtypes. They looked for mutations also using 10 kb bins, binning the genome in 279094 bins and finding only 91343 carrying mutations; therefore, they only found mutations 32% of the time (25, 28). In contrast to these studies, the present work shows the development of a tool to analyze the DNA repair function, focusing on memory or activated B-cell switch regions making the infrequent event of somatic "mutational "break repair as frequent as one event per read.

### DNA repair analysis by SWIBRID has the potential to identify immune-related diseases:

Analysis of DNA repair function provides information about a patient's chance of repairing a break error-free compared to a healthy sample. Besides cancer, DNA repair malfunction is associated with the development of neurological diseases. For instance, mutations in ataxia-telangiectasia (ATM) can lead to the development of ataxia-telangiectasia, a neurodegenerative disease that causes ataxia early in life. DNA repair analysis also focuses on screening the function of DNA repair proteins, even though many DNA repair factors remain unknown. Unlike present SNP screening methods, the DNA repair biomarker could potentially predict neurodevelopmental diseases associated with DNA stress early in life.

On the other hand, T- and B-cell diversification rely on the imprecise gene recombination of their T-cell receptors (TCR) and B-cell receptors (BCR). Gene recombination requires a mutational DNA repair mechanism such as cNHEJ to repair the joints. It is logical to assume that impairment of DNA repair proteins in humans can result in primary immunodeficiencies. In fact, Ligase 4 deficiency is the cause of Ligase IV Syndrome, a T-B-Natural Killer (NK)+ severe combined immunodeficiency. The diagnosis of B-cell-related primary immunodeficiencies includes the analysis of clinical signs, such as recurrent infections or antibody classes in the blood. Genomic screening is not often used as diagnostics, given that nuclear factor kappa B subunit 1 *(NFKB1),* the most common defect causing common variable immunodeficiency (CVID), is only found in 4% of the patients. Thaventhiran et al., 2020 performed a Genome-wide association studies (GWAS) on 974 patients carrying sporadic or familiar CVID with a large control dataset in pursuit of novel common genomic defects. Unfortunately, they did not find a CVID genomic defect more common than *NFKB1,* but they found new ones that the Exome Aggregation Consortium did not cover, such as a deletion on Actin Related Protein 2/3 Complex Subunit 1B (*ARPC1B)* that impaired the assembly of actin in immune cells (30). The research provides evidence that undiscovered genetic variants could facilitate accurate primary B-cell immunodeficiencies diagnosis. However, genomic screenings for CVID would be costly to be considered in clinical diagnostics. Therefore, the phenotypic analysis of switch-joints may be preferable over genotyping for diagnosis. It would be of utmost importance to detect them early in life, as some primary immunodeficiencies are not expressed symptomatically until adulthood, threatening the life of the patients with the development of disease complications (31). SWIBRID analyzes the switch-joints of B-cells and can observe impaired B-cell diversification or switching - two leading causes of B-cell primary immunodeficiencies (according to European Society for Immunodeficiencies Registry) - likely allowing their diagnosis in early life.

In conclusion, the method of the invention has the potential to identify neurodevelopmental diseases and immunodeficiencies by switch-joints analysis.

### SWIBRID assesses DNA repair based on the architecture of switch-joints considering available and new features:

The study of CSR by switch-joint analysis started in 1991. SM-SE switch-joints were PCR amplified to disprove that IgE expression occurred due to RNA splicing (32). Since then, switch-joints have been used for B-cell-related studies and exploited as a tool to study the mechanisms of DNA repair. For example, switch-joints were used to discover that the combination of SHLD1 and Xlf1 was crucial for the inhibition of resection (7) or to confirm that staggered dsDNA breaks are commonly repaired using resection and microhomology (33). However, switch-joint analysis is commonly standardized by the total number of reads or not at all. Standardizing the switch-joint analysis using B-cell clones is important. It is also done in VDJ-sequencing analysis. For instance, somatic hypermutation analysis in VDJ-clones is standardized per unique B-cell clones to avoid overestimating events in samples with low diversity (34, 35). By developing SWIBRID, the inventors have created a tool that not only studies the DNA repair of breaks during CSR but also quantifies the number of individual B-cell clones in a sample, making possible for the first time the standardization of DNA repair analysis using switch-joints.

The analysis of the breakpoint profile in switch-joint studies the homology used during the repair, a feature that can help determine the DNA repair mechanism involved (36). The results of the method of the invention show that in CH12, a higher homology usage was only observed for Rif1^{-/-} (Fig. 7F), which agrees with the fact that Rif1 silencing in HeLa cells promotes HR (39) because Rif1 directly inhibits HR by competing with BRCA1 (*40, 41*)*.*

Inversions were introduced as a hallmark of deficient CSR in 2015 since they rarely occurred in healthy samples, but upon cNHEJ protein depletion, they became more prone (10). The inventors found that inversions occurred at a median frequency of one per eff_ncluster in WT CH12 (Fig. 7G). However, Ligase 4^{-/-} CH12 mean inversions per eff_nclusters was 25.8. It was shown that XRCC4^{-/-}mice, protein forming a heterodimer with Ligase 4 during cNHEJ, have increased inversional CSR joints in SM-SG1 and SM-SE (42). The results could be explained by the fact that XRCC4/Ligase 4 heterodimer is necessary to limit the formation of inversional CSR joints. (7, 10, 43, 44)(45)

SWIBRID includes new features to help the analysis of DNA repair during CSR, such as eff_nclusters. In the past, switch-joint analysis has been standardized by the number of reads (7, 10). However, standardizing by the number of reads is incorrect because clones in a polyclonal library are represented by one or more reads. For instance, all CH12 knock-outs, especially Rif1^{-/-}, presented lesser diversity than WT CH12 (Fig. 6B-3). Analysis standardized using reads would have given different results. For example, the inversion frequency in Rif1^{-/-} CH12 standardized using reads would be 0.0044, and correctly doing it with eff_nclusters is 2.81. The absence of DNA repair proteins results in DNA repair deficiency, leading to an inefficient CSR and lower B-cell diversity. Using SWIBRID, the inventors analyzed the switch-joints generated by PacBio of Vincendeau et al., 2022. They observed that even though they standardized their data using the number of reads, they had fewer unique B-cell clusters (e.g., the WT sample contained 1276 reads and only 174 unique B-cell clones). Thus, SWIBRID has a significant advantage of standardizing the data in a high-throughput manner by the number of individual B-cell clones, which is particularly important when analyzing samples with different amounts of B-cell diversity.

Other features that the inventors classified are single, multiple and within breakpoints that differentiate between breakpoints that result in productive CSR, sequential switching or intra-switch deletions, respectively (Fig. 7A). DNA repair deficient cells are prone to leaving DNA breaks unrepaired and entering apoptosis. Therefore, it is expected to see fewer DNA breaks repaired occurring in DNA repair deficiencies. Productive CSR can result from a minimum of one "single breakpoint", between SM and any other switch region. Multiple and within breakpoints are those repair events occurring after sequential switching and deletions within the same switch region, respectively. These breakpoints are not essential to generate a productive CSR and most likely occur after a single breakpoint occurs. The results in CH12 show that multiple and within breakpoints are less frequent in DNA repair deficiencies than in WT samples. This suggests that DNA repair deficient B-cells have a higher chance of surviving when they experience fewer DNA breaks.

In conclusion, SWIBRID is a tool that uses switch-joints to study DNA repair during CSR using known features, namely, homology and inversions, and novel features, namely, eff_nclusters and classified breakpoints. SWIBRID is crucial to analyze samples with different degrees of diversity since the standardization by eff_nclusters weights features among the samples equally.

### CH12 knockout switch-joint features agree with the literature and unravel new features:

53BP1 is a protein known to work with Rif1 to promote cNHEJ (*41*), but it has also been related to homologous recombination during G2 phase(56). 53BP1^{-/-} CH12 has been previously observed to switch mainly to IgA and almost not to IgG1 or IgG2b (7, 57). This agrees with the almost exclusive SM-SA switch-joints observed in our results in 53BP1^{-/-} CH12. Also, it could explain the low amount of eff_nclusters found in 53BP1^{-/-} CH12 since the lack of class-switching to other isotypes reduces the possibilities of generating a variety of B-cell clones. Also, 53BP1^{-/-} had been shown to have increased breakpoints in the IGH locus (57); however, we observed a lower amount of within breakpoints. Unfortunately, the methodology used to calculate the breakpoints in 53BP1^{-/-} in Dev et al., 2015 was not explained, making the analysis comparison less veridic. Therefore, SWIBRID data could replicate the results previously observed in 53BP1^{-/-} CH12.

BRCA1 is a protein involved in homology-directed DNA repair, which is not essential during CSR but helps to repair long-lasting CSR breaks during the S phase (58). The inventors observed that switch-joint features in BRCA1^{-/-} CH12 were similar to WT, except for the single breakpoints, where BRCA1^{-/-} CH12 had the highest values from all genotypes (Fig 7C). A high amount of single breakpoints indicates a higher diversity of break positions in the switch-joints. One assumption is that a large number of breaks would give a higher spread value in SM and SA. Spread indicates the distance between the most upstream and downstream breakpoints occurring in a switch region. However, SM and SA spread in BRCA1^{-/-} CH12 was not significantly different from WT CH12. There was only a slightly higher tendency of BRCA1^{-/-} CH12 SM spread than WT CH12 (Fig. 7H). Interestingly, lack of BRCA1 has been observed not to affect the number of switch-joints(59), agreeing with the present results where it can be observed that this deficiency and WT have similar eff_nclusters.

Ligase 4 is the enzyme that, in complex with XRCC4, end-ligates two dsDNA break ends and finalizes cNHEJ (60). As previously outlined, Ligase 4^{-/-} CH12 switch-joints presented a high occurrence of inversions. Also, Ligase 4^{-/-} CH12 showed a high SG3 spread (Fig. 7H). There is so far no explanation for the high spread in SG3. However, dsDNA break could be processed by aEJ (61) due to Ligase 4 loss, suffering resection in both directions in an attempt to find homology and repair the DNA break. SM and SA could experience a lower resection because homology between them can be found earlier; however, SGs are less homologous to SM than SA.

Rif1 protects dsDNA breaks from resection during DNA repair and replication fork stalling (41, 62). Rif1 loss impairs cNHEJ, the preferred DNA repair during CSR, promoting homology-directed repair (41, 63). The present results show that Rif1^{-/-} CH12 uses the highest 4-mer homology of all samples, which would agree that homology-directed DNA repair has repaired the breaks in Rif1^{-/-} CH12 knockout. Rif1^{-/-} CH12 had a very low spread in all switch regions (Fig. 7H) and the lowest amount of eff_clusters (2-fold less than the second lower, 53BP1^{-/-}). These features depend on each other since every new eff_ncluster provides new breakpoint information for the spread unit. The low number of eff_nclusters could be explained by the lack of cNHEJ (Fig. 6B-3). It is important to note that Rif1^{-/-}CH12 replicates gave rise to many reads per cluster, which could mean that upon activation, most of the Rif1^{-/-} clones died, and the few successfully class-switched B-cells, proliferated and occupied the majority of the culture.

Resection is important in understanding the DNA repair pathway repairing a break, in particular, to understand the role of 53BP1, (64) Rif1 (40) and BRCA1 ;(65) considering the two first proteins inhibit resection while the latter promotes it. Resection was not required to separate the genotypes in the PCA; however, the analysis could benefit from having such a feature. SWIBRID could adopt the method that Vincendeau et al., 2022 followed to measure resection by measuring the length of the amplicons. Longer amplicons would relate to less resection and vice versa.

In conclusion, CH12 knockout switch-joint features analyzed by SWIBRID agree with the literature, making the present results robust. New features of the genotypes, such as higher diversity of single breakpoints in BRCA1^{-/-} and low amount of eff_clusters in Rif1^{-/-} show how SWIBRID switch-joint analysis can open a new way to analyze DNA repair during CSR discovering new features.

### The method of the invention as a diagnostic tool based on specific disease cohorts with age- and gender-matching donors:

The method of the invention, SWIBRID, or the DNA break repair footprint generated by it can become a diagnostic tool used in the clinic to predict diseases. The diseases it will be able to predict are those caused by deficiencies in DNA repair, in particular, ds DNA break repair, or B-cell biology. Thus, cancer, neurodevelopmental diseases, autoimmune diseases or immunodeficiencies would be the main target of the method of the invention. Cohorts of different diseases with age- and gender-matching healthy controls would allow a patient sample to be tested against several diseases (Fig. 9). These cohorts might be challenging to obtain because i) diseases are rare, such as CVID, which occurs in 1:25000 people (90) or ii) the life expectancy of diseases is low, like Cockayne syndrome, whose patients live up to 16 years old, (91).

Therefore, cohorts could also be accomplished by silencing specific proteins in HD (Fig.9). This approach would generate a cohort of sick and healthy matching donors, where the sick donors would be the healthy samples with protein-silenced B-cells that would be activated *in vitro*(*92*)*.* It would be helpful to consider that the silenced B-cells would be activated *in vitro* and not *in vivo.* Considering that *in vitro* and *in vivo* activation do not elicit the same results, (3, 93) this issue should be addressed by comparing the *in vitro* activation with the *in vivo* switch-joint of the donors using their CD27⁺IgG/A⁺IgM⁻ B-cells.

Healthy donors used for control can be subjects that never had cancer. However, to improve results still more, it may be confirmed that they are also healthy in terms of mutations in proteins. Thus, exome sequencing may be performed to screen for potentially damaging mutations that will affect the SWIBRID analysis.

In conclusion, the DNA break repair footprint provides a prognostic disease tool that allows for comparison with several disease cohorts. The generation of disease-specific cohorts with age- and gender-matching healthy controls confirmed by exome sequencing will further improve results. Rare and low-life expectancy disease cohorts could be obtained by silencing specific B-cell proteins from healthy donors.

### Ovarian cancer (OC) SWIBRID experiment

20 blinded ovarian cancer samples, 10 of these BRCA-mutated, and 10 BRCA wildtype and 20 women age-matched (up to +/- 5 years) healthy donors are anaylsed. A barcoded switch PCR using SM forward, SG reverse and SA reverse primers of SEQ ID NO: 1-3 is carried out. The samples are sequenced, 20 samples per run, using a MinION^{®} sequencer.

A PCA analysis can be carried out. It is expected to separate OC samples from healthy donor samples. It is also expected that only OC samples will show different clusters, differentiating, e.g., between BRCA mutated or HPV driven or spontaneous cancer.

Exome sequencing may allow to determine: i) if potential healthy donors clustering with OC have any DNA repair protein defects, ii) OC BRCA-WT samples clustering with BRCA mutated samples have Homologous Recombination defects. When sufficient numbers of samples have been analyzed, machine learning can be used to classify further samples, e.g., to identify OC cancers versus healthy donors.

### Literature

1. E. Y. Lee, S. Betschel, E. Grunebaum, Allergy Asthma Clin Immunol. 18, 21 (2022).
2. J. M. Routes, J. W. Verbsky, Pediatr Clin N Am. 64, 27-37 (2017).
3. M. Lebedin et al., Proc National Acad Sci. 119 (2022), doi:10.1073/pnas.2205470119.
4. J. Hu et al., Nat Protoc. 11, 853-871 (2016).
5. J. Bruijnesteijn, M. van der Wiel, N. G. de Groot, R. E. Bontrop, Front Immunol. 12, 722181 (2021).
6. T. T. Wu, M. Reid-Miller, H. M. Perry, E. A. Kabat, Embo J. 3, 2033-2040 (1984).
7. E. Vincendeau et al., Nat Commun. 13, 3707 (2022).
8. K. Pieper et al., Nature. 548, 597 (2017).
9. J. Tan et al., Nature. 529, 105 (2016).
10. J. Dong et al., Nature. 525, 134-139 (2015).
11. S. L. Noir et al., Plos Genet. 17, e1009288 (2021).
12. X. S. Wang et al., Proc National Acad Sci. 117, 25700-25711 (2020).
13. F. Boyer et al., J Immunol. 198, 4148-4155 (2017).
14. H. Jia, Y. Guo, W. Zhao, K. Wang, Sci Rep-uk. 4, 5737 (2014).
15. J. Stavnezer, J. E. J. Guikema, C. E. Schrader, Annu Rev Immunol. 26, 261-292 (2008).
16. H. Xiong, J. Dolpady, M. Wabl, M. A. C. de Lafaille, J. J. Lafaille, J Exp Medicine. 209, 353-364 (2012).
17. R. J. M. Bashford-Rogers et al., Nature. 574, 122-126 (2019).
18. M. A. Turchaninova et al., NatProtoc. 11, 1599-1616 (2016).
19. M. Nakamura et al., Int Immunol. 8, 193-201 (1996).
20. A. Reboldi et al., Science. 352, aaf4822 (2016).
21. R. Shinkura et al., Nat Immunol. 5, 707-712 (2004).
22. E. Enervald et al., J Exp Medicine. 210, 2503-2513 (2013).
23. C. Mendez-Dorantes, R. Bhargava, J. M. Stark, Gene Dev. 32, 524-536 (2018).
24. T. A. Knijnenburg et al., Cell Reports. 23, 239-254.e6 (2018).
25. M. A. Sherman et al., Nat Biotechnol, 1-10 (2022).
26. Y. Wang et al., J Recept Sig Transd. 41, 304-311 (2021).
27. M. A. Boti, P. G. Adamopoulos, P. Tsiakanikas, A. Scorilas, Genes-basel. 12, 839 (2021).
28. H. E. Machado et al., Nature. 608, 724-732 (2022).
29. J. Ganz et al., Biorxiv Prepr Serv Biology (2023), doi:10.1101/2023.01.14.523958.
30. J. E. D. Thaventhiran et al., Nature. 583, 90-95 (2020).
31. J. Routes et al., J Clin Immunol. 34, 398-424 (2014).
32. S. K. Shapira et al., Proc National Acad Sci. 88, 7528-7532 (1991).
33. A. K. Ling et al., Proc National Acad Sci. 115, 201720962 (2018).
34. H. iJspeert et al., Front Immunol. 7, 410 (2016).
35. N. Nouri, S. H. Kleinstein, Plos Comput Biol. 16, e1007977 (2020).
36. T. Saha, D. Sundaravinayagam, M. D. Virgilio, Trends Biochem Sci. 46, 184-199 (2020).
37. J. M. Lumsden et al., J Exp Medicine. 200, 1111-1121 (2004).
38. S. Saito, R. Maeda, N. Adachi, Nat Commun. 8, 16112 (2017).
39. S.-Y. Isobe, K. Nagao, N. Nozaki, H. Kimura, C. Obuse, Cell Reports. 20, 297-307 (2017).
40. M. D. Virgilio et al., Science. 339, 711-715 (2013).
41. L. Feng, K.-W. Fong, J. Wang, W. Wang, J. Chen, J Biol Chem. 288, 11135-11143 (2013).
42. J. L. Crowe et al., Proc National Acad Sci. 117, 22953-22961 (2020).
43. X. Xie et al., Embo J. 41, e109324 (2022).
44. X. Liu et al., Cell Res. 30, 732-744 (2020).
45. Q. Pan-Hammarström, Y. Zhao, L. Hammarström, Adv Immunol. 93, 1-61 (2007).
46. D. D. Dudley, J. Chaudhuri, C. H. Bassing, F. W. Alt, Adv Immunol. 86, 43-112 (2005).
47. N. P. Keegan, S. D. Wilton, S. Fletcher, Frontiers Genetics. 12, 806946 (2022).
48. J. Ouyang et al., Brit J Cancer. 126, 1113-1124 (2022).
49. J. O'Brien, H. Hayder, Y. Zayed, C. Peng, Front Endocrinol. 9, 402 (2018).
50. A. Kozomara, M. Birgaoanu, S. Griffiths-Jones, Nucleic Acids Res. 47, gky1141- (2018).
51. B. H. R. D. Fonseca, D. S. Domingues, A. R. Paschoal, Bioinformatics. 35, btz153 (2019).
52. P. P. Amaral, M. B. Clark, D. K. Gascoigne, M. E. Dinger, J. S. Mattick, Nucleic Acids Res. 39, D146-D151 (2011).
53. P. J. Hilleren, R. Parker, Mol Cell. 12, 1453-1465 (2003).
54. L. K. Lerner et al., Front Immunol. 13, 871766 (2022).
55. A. J. Matthews, S. Zheng, L. J. DiMenna, J. Chaudhuri, Adv Immunol. 122, 1-57 (2014).
56. A. Kakarougkas et al., Nucleic Acids Res. 41, 9719-9731 (2013).
57. H. Dev et al., Nat Cell Biol. 20, 954-965 (2018).
58. A. Yamane et al., Cell Reports. 3, 138-147 (2013).
59. A. Björkman et al., Proc National Acad Sci. 112, 2157-2162 (2015).
60. Q. Pan-Hammarström et al., J Exp Medicine. 201, 189-194 (2005).
61. N. J. Goff et al., Nucleic Acids Res. 50, 11058-11071 (2022).
62. A. R. Chaudhuri et al., Nature. 535, 382-387 (2016).
63. J. R. Chapman et al., Mol Cell. 49, 858-871 (2013).
64. A. Bothmer et al., J Exp Med. 207, 855-865 (2010).
65. A. Cruz-Garcia, A. López-Saavedra, P. Huertas, Cell Reports. 9, 451-459 (2014).
66. C. Scheepers et al., Cell Reports. 33, 108430 (2020).
67. K. Kitaura et al., Front Immunol. 8, 389 (2017).
68. R. Levin-Klein, Y. Bergman, Front Immunol. 5, 625 (2014).
69. J.-M. Lambert, M. O. Ashi, N. Srour, L. Delpy, J. Saulière, Int J Mol Sci. 21, 1335 (2020).
70. D. Lindholm, L. Korhonen, O. Eriksson, S. Kõks, Frontiers Cell Dev Biology. 5, 48 (2017).
71. M. Rose, J. T. Burgess, K. O'Byrne, D. J. Richard, E. Bolderson, Frontiers Cell Dev Biology. 8, 564601 (2020).
72. S. Camero et al., J Cancer Res Clin. 145, 137-152 (2019).
73. P. Gralewska, A. Gajek, A. Marczak, A. Rogalska, Int J Mol Sci. 22, 10557 (2021).
74. E. K. Lee, U. A. Matulonis, Cancers. 12, 2054 (2020).
75. R. Bhargava, D. O. Onyango, J. M. Stark, Trends Genet. 32, 566-575 (2016).
76. J. H. Barlow et al., Cell. 152, 620-632 (2013).
77. E. M. Cortizas et al., J Exp Medicine. 213, 2459-2472 (2016).
78. D. Menolfi, S. Zha, Cell Biosci. 10, 8 (2020).
79. T. C. Nepomuceno et al., Int J Mol Sci. 18, 1886 (2017).
80. M. Muramatsu et al., Cell. 102, 553-563 (2000).
81. J. M. Daley, P. Sung, Mol Cell Biol. 34, 1380-1388 (2014).
82. A. Piazza et al., Nat Cell Biol. 23, 1176-1186 (2021).
83. K. Kumazaki et al., J Immunol. 178, 2192-2203 (2007).
84. B. Schwer et al., Proc National Acad Sci. 113, 2258-2263 (2016).
85. N. Michel et al., Sci Rep-uk. 12, 12156 (2022).
86. I. Rybanska-Spaeder et al., Mol Cancer Res. 11, 1223-1234 (2013).
87. G. Balmus et al., Nat Commun. 10, 87 (2019).
88. A. T. S. Boone et al., Frontiers Pediatrics. 6, 426 (2019).
89. D. Simsek, M. Jasin, Nat Struct Mol Biol. 17, 410-416 (2010).
90. P. Tuijnenburg et al., J Allergy Clin Immun. 142, 1285-1296 (2018).
91. L. E. Brace et al., Aging Cell. 12, 1144-1147 (2013).
92. T. Shih, S. De, B. J. Barnes, Front Immunol. 10, 1652 (2019).
93. T. Nojima et al., Nat Commun. 2, 465 (2011).

## Claims

1. A method for determining a DNA break repair deficiency of a subject, comprising steps of
a) providing a sample from the subject comprising B-cells;
b) analyzing switch-joints of the IGH locus of said B-cells, the analysis comprising sequencing the switch-joints using long-read sequencing to generate a library of switch-joint sequencing reads;
c) analyzing the library to determine the DNA break repair footprint and determine a DNA break repair deficiency.

2. The method of claim 1, wherein in step c), a number of individual B-cell clones in the sample is determined by clustering of sequencing reads, and the number of clusters is considered to represent the number of individual B-cell clones in the sample,
preferably, wherein only the smallest number of clusters that contain only 90-99% of reads are further analyzed, and the number of these clusters is considered to represent the number of effective individual B-cell clones in the sample.

3. The method of claim 2, wherein in step c), alignments of reads to a reference genome are transformed into coverage patterns which are clustered, preferably, using hierarchical agglomerative clustering.

4. The method of any of claims 2 or 3, wherein similarities between coverage patterns for different reads are analyzed to provide clusters, optionally based on Jaccard or Cosine distance, preferably, Cosine distance,
wherein, optionally, gaps in the alignment smaller than 75 bp are ignored.

5. The method of any of the preceding claims, wherein the analysis of step c) comprises using machine learning to classify a pattern of features, preferably, features derived from clustered read coverage patterns,
wherein optionally, at least 9, preferably, at least 10 features are selected from the group comprising:
| Donor_score_TCCA |
|---|
| eff_nclusters |
| donor_score_TG |
| donor_score_GC |
| donor_score_AT |
| mean_length |
| donor_score_CAGCT |
| frac_SA |
| donor_score_CA |
| mean_GC |
| num_mutated_pos |
| SM_downstream_bias |
| receiver_score_CA |
| cluster_inverse_simpson |
| donor_score_CAGCC |
| std_length |
| spread_SA |
| receiver_score_TG |
| log10_inversions |
| log10_nreads |

6. The method of any of the preceding claims, wherein in step c), the analysis comprises provision of a two-dimensional breakpoint histogram showing the frequency of switch-joints between donor and acceptor with donor switch region coordinates on one axis and acceptor switch region coordinates on the other axis, wherein, optionally, machine learning is carried out considering every position in the two-dimensional breakpoint histogram as a feature.

7. The method of any of the preceding claims, wherein in step c), breakpoints of switch-joints, inserts and isotypes are identified.

8. The method of any of the preceding claims, wherein in step c) sequencing reads are filtered and reads not meeting quality criteria are discarded, wherein at least 3, preferably at least 4, at least 5 or all of the following quality criteria apply:
i. Reads are longer than 500 nt;
ii. The sequence of the read must contain at least a 20 nucleotide sequence of one switch region;
iii. The sequence of the read must align in more than 90% of its length to the genome, and/or,
iv. Reads must follow B-cell class switch rules;
and/or, in case the analysis of step b) comprises, before sequencing, amplification of the switch-joints by PCR,
v. Reads contain a SM forward primer sequence and a reverse primer sequence within 100 nt from the beginning and the end of the read, respectively; wherein the primers preferably have SEQ ID NO: 1, 2 and 3; and/or
vi. Reads should not contain single primers outside 100 nt from the beginning or the end of the read.

9. The method of any of the preceding claims, wherein the analysis of step b) comprises, before sequencing, amplification of the switch-joints, by a method selected from the group comprising PCR and LAM-HTGTS (linear amplification mediated high-throughput genomic translocations sequencing), preferably, PCR.

10. The method of claim 9, wherein the amplification is a PCR using a forward primer annealing to DNA 5' to the switch µ (SM) region and at least one reverse primer annealing to DNA 3' to a switch γ1, γ 2, γ3 and/ or γ4 (SG1, SG2, SG3, SG4) region and/or 3' the switch α1 (SA1) and/or switch α 2 (SA2) region.

11. The method of claim 10, wherein the subject is a human and three primers are used:
a) a forward primer annealing to DNA 5' to the SM region, wherein, optionally, the primer has SEQ ID NO: 1;
b) a reverse primer annealing to DNA 3' to the SG1, SG2, SG3 and SG4 regions, wherein, optionally, the primer has SEQ ID NO: 3; and,
c) a reverse primer annealing to DNA 3' to the SA1 and SA2 region, wherein, optionally, the primer has SEQ ID NO: 2.

12. The method of any of claims 1-8, wherein the analysis of step b) comprises, before sequencing, Cas9 cleavage of DNA at least 5' to the SM region, and optionally, in addition 3' of a SG1, SG2, SG3 and/or SG4 region and/or SA1 and/or SA2 region.

13. The method of any of the preceding claims, wherein the sequencing is performed by a third generation sequencing method selected from the group comprising
a) nanopore sequencing such as MinION^{®} sequencing and
b) single-molecule real-time (SMRT) sequencing such as PacBio^{®} sequencing, preferably, nanopore sequencing.

14. A method of any of the preceding claims, comprising comparing the DNA-break repair footprint of the subject with the DNA-break repair footprint of at least one control sample having a deficiency in the DNA-break repair machinery, wherein the deficiency is selected from the group comprising Activation-induced cytidine deaminase (AID) deficiency, Ataxia-telangiectasia mutated (ATM) deficiency, Breast cancer gene 1 (BRCA1) deficiency, Breast cancer gene 2 (BRCA2) deficiency, Ligase 4 deficiency, RAD51 Recombinase paralog C (RAD51C) deficiency, RAD51 Recombinase paralog D (RAD51D) deficiency, Partner and localizer of BRCA2 (PALB2) deficiency, BRCA1 Associated RING Domain 1 (BARD1) deficiency, excision repair cross complementation group 1 (ERCC1) deficiency, Artemis deficiency, recombination-activating gene (RAG) deficiency, DNA-dependent protein kinase, catalytic subunit (DNA-PKcs) deficiency, Phosphatase and tensin homolog (PTEN) deficiency, X-ray repair cross-complementing protein 4 (XRCC4) deficiency, X-ray repair cross-complementing protein 2 (XRCC2) deficiency, X-ray repair cross-complementing protein 1 (XRCC1) deficiency, DNA polymerase δ deficiency, Ligase 1 deficiency, Ligase 3 deficiency, XRCC4-like factor (XLF) deficiency, ERCC excision repair 6 like 2 (ERCC6L2) deficiency, Proliferating cell nuclear antigen (PCNA) deficiency, Lynch syndrome, p53 binding protein 1 (53BP1) deficiency and NIPBL deficiency, wherein preferably, the deficiency is in the dsDNA break repair machinery.

15. A method of predicting DNA repair efficiency in a subject, comprising carrying out the method of any of the preceding claims, wherein, optionally, DNA repair efficiency in a cell that is not a B-cell is predicted.

16. A method for determining the risk to develop a cancer, an immunodeficiency such as common variable immunodeficiency (CVID) or a neurodevelopmental disease such as ataxia telangiectasia (AT), or for determining the prognosis of a cancer, comprising carrying out the method of any of the preceding claims, wherein preferably, the method determines the risk to develop a cancer, wherein, optionally, the cancer is not a B-cell lymphoma, or
a method for selecting a method of treatment of a disease selected from the group comprising a cancer, an immunodeficiency such as CVID or a neurodevelopmental disease such as AT, comprising carrying out the method of any of the preceding claims, and selecting an appropriate treatment based on the DNA-break repair profile of the subject.

17. A computer program product comprising instructions which, when the program is executed by a computer, cause the computer to carry out analysis of step c) of the method of any of claims 1-16, wherein the computer-implemented program comprises instructions to
a) filter reads according to claim 8,
b) cluster sequences to identify effective B-cell clones according to any of claims 2-7,
c) derive aggregated features from clustered read coverage,
c) use machine learning to classify these features,
wherein preferably, at least 9 features are selected from the group comprising:
| Donor_score_TCCA |
|---|
| eff_nclusters |
| donor_score_TG |
| donor_score_GC |
| donor_score_AT |
| mean_length |
| donor_score_CAGCT |
| frac_SA |
| donor_score_CA |
| mean_GC |
| num_mutated_pos |
| SM_downstream_bias |
| receiver_score_CA |
| cluster_inverse_simpson |
| donor_score_CAGCC |
| std_length |
| spread_SA |
| receiver_score_TG |
| log10_inversions |
| log10_nreads |
, or a kit suitable for carrying out the method of any of claims 1-16, the kit comprising a computer-readable medium comprising said computer-implemented program and the primers of claim 11, preferably, primers of SEQ ID NO: 1, 2 and 3, optionally, wherein either the forward or the reverse primers further comprises a barcode sequence.
